# EUROPEAN PATENT SPECIFICATION

(11) **EP 2 318 042 B1**
(45) Date of publication and mention of the grant of the patent: **20.08.2014**
(21) Application number: 09781248.1
(22) Date of filing: 29.07.2009
(51) Int. Cl.: A61K 39/12, A61P 35/00

(54) **VACCINE AGAINST HPV**
IMPFSTOFF GEGEN HPV
VACCIN CONTRE HPV

(30) Priority: 31.07.2008 US 85101 P; 08.05.2009 US 176561 P
(43) Date of publication of application: 11.05.2011
(73) Proprietor: GlaxoSmithKline Biologicals S.A., 1330 Rixensart (BE)
(72) Inventor: DESCAMPS, Dominique, B-1330 Rixensart (BE); GIANNINI, Sandra, B-1330 Rixensart (BE); LECRENIER, Nicolas, B-1330 Rixensart (BE); STEPHENNE, Jean, B-1330 Rixensart (BE); WETTENDORFF, Martine Anne Cecile, B-1330 Wavre (BE)
(74) Representative: Dalton, Marcus Jonathan William
(86) International application number: PCT/EP2009/059820
(87) International publication number: WO 2010/012780

(56) References cited:
- WO-A-2005/123125
- WO-A2-01/17551
- US-A1- 2007 014 810
- CUTTS F T ET AL: "Human papillomavirus and HPV vaccines: a review." BULLETIN OF THE WORLD HEALTH ORGANIZATION SEP 2007, vol. 85, no. 9, September 2007 (2007-09), pages 719-726, XP002550641 ISSN: 0042-9686
- HERRERO R ET AL: "Rationale and design of a community-based double-blind randomized clinical trial of an HPV 16 and 18 vaccine in Guanacaste, Costa Rica" VACCINE 20080902 GB, vol. 26, no. 37, 18 July 2008 (2008-07-18) , pages 4795-4808, XP002550642 ISSN: 0264-410X
- HARPER DIANE M ET AL: "Efficacy of a bivalent L1 virus-like particle vaccine in prevention of infection with human papillomavirus types 16 and 18 in young women: a randomised controlled trial." LANCET 2004 NOV 13-19, vol. 364, no. 9447, 13 November 2004 (2004-11-13), pages 1757-1765, XP002550643 ISSN: 1474-547X
- BRYAN JANINE T: "Developing an HPV vaccine to prevent cervical cancer and genital warts." VACCINE 20 APR 2007, vol. 25, no. 16, 20 April 2007 (2007-04-20), pages 3001-3006, XP002559704 ISSN: 0264-410X
- "PACKAGE LEAFLET: INFORMATION FOR THE USER. GARDASIL, SUSPENSION FOR INJECTION IN A PRE-FILLED SYRINGE" 2006, pages 48-54, XP002559705 Retrieved from the Internet: URL:https://www.ebs.tga.gov.au/servlet/xml millr6?dbid=ebs/PublicHTML/pdfStore.nsf&do cid=57A5F84BB7066081CA2574DD0008D861&agid= (PrintDetailsPublic)&actionid=1> [retrieved on 2009-12-07]
- SANOFI PASTEUR MSD LIMITED: "GARDASIL-SUMMARY OR PRODUCT CHARACTERISTICS" 20 September 2006 (2006-09-20), pages 1-16, XP002559706 Retrieved from the Internet: URL:http://emc.medicines.org.uk/document.a spx?documentId=19016> [retrieved on 2009-12-11]

## Description

### Field of the Invention

The present invention relates to human papillomavirus (HPV) vaccines.

### Background of the Invention

Gardasil™ (Merck & Co Inc) is an HPV vaccine comprising an HPV 6 virus-like particle (VLP) consisting of an HPV 6 L1 protein, an HPV 11 VLP consisting of an HPV 11 L1 protein, an HPV 16 VLP consisting of an HPV 16 L1 protein, and an HPV 18 VLP consisting of an HPV 18 L1 protein, and an aluminium adjuvant. The VLPs are present in an amount of 20 µg, 40 µg, 40 µg, and 20 µg, respectively, per dose. The vaccine is administered as a 3-dose regimen according to a 0, 2, 6 month schedule.

Cervarix™ (GlaxoSmithKline) is an HPV vaccine comprising an HPV 16 VLP consisting of an HPV 16 L1 protein, and an HPV 18 VLP consisting of an HPV 18 L1 protein, and an adjuvant containing aluminium hydroxide and 3-desacyl-4'-monophosphoryl lipid A, also referred to as 3D-MPL. The VLPs are present in an amount of 20 µg each per dose. 3D-MPL is present in an amount of 50 µg per dose. This vaccine is also administered as a 3-dose regimen according to a 0, 1, 6 month schedule.

Reference is made to for example Bulletin of the World Health Organization, vol. 85, 2007, 719-726.

### Summary of the invention

The present invention is an improved HPV vaccine that is effective when administered as a 2-dose regimen.

Accordingly, the present invention relates to:
1. A composition comprising HPV 16 and HPV 18 virus like particles (VLPs) together with a pharmaceutically acceptable excipient, for use as a vaccine in the prevention of human papillomavirus related disease or infection in a subject wherein the vaccine is formulated for administration to a subject 14 years of age or below according to a two dose regimen consisting of a first dose and a second dose.
2. A composition for use according to item 1 wherein the second dose is to be administered 2 or 3 months after the first dose.
3. A composition for use according to item 1 wherein the second dose is to be administered more than two months after the first dose.
4. A composition for use according to item 3 wherein the second dose is to be administered 6 months after the first dose.
5. A composition for use according to any of items 1 to 4 wherein the vaccine further comprises an adjuvant.
6. A composition for use according to item 5 wherein the adjuvant comprises an aluminium salt.
7. A composition for use according to item 6 wherein the aluminium salt is aluminium hydroxide.
8. A composition for use according to any of items 5 to 7 wherein the adjuvant comprises a lipid A derivative.
9. A composition for use according to item 8 wherein the adjuvant comprises 3D-MPL and aluminium hydroxide.
10. A composition for use according to any preceding item wherein the 2 doses each comprise more than 20 µg of each of HPV 16 VLPs and HPV 18 VLPs, or 20 µg or 40 µg of each of HPV 16 VLPs and HPV 18 VLPs.
11. A composition for use according to any preceding item wherein the VLPs comprise L1.
12. A composition for use according to any preceding item wherein only VLPs of HPV types 16 and 18 are present in the vaccine.
13. A composition for use according to any preceding item wherein the vaccine is for the prevention of human papillomavirus related disease or infection in females.

Further disclosed is a method for the manufacture of a vaccine, the method comprising a) combining HPV 16 VLPs, HPV 18 VLPs and an adjuvant to form a vaccine, and b) filling storage or delivery vessels with a human dose of the vaccine containing greater than 20 µg of HPV 16 VLPs and greater than 20 µg of HPV 18 VLPs.

Further disclosed is a method for the manufacture of a vaccine, the method comprising a) combining HPV 16 VLPs, HPV 18 VLPs and an adjuvant to form a vaccine, and b) filling storage or delivery vessels with a human dose of the vaccine containing 30 µg of HPV 16 VLPs and 30 µg of HPV 18 VLPs.

Further disclosed is a method for the manufacture of a vaccine, the method comprising a) combining HPV 16 VLPs, HPV 18 VLPs and an adjuvant to form a vaccine, and b) filling storage or delivery vessels with a human dose of the vaccine containing 40 µg of HPV 16 VLPs and 40 µg of HPV 18 VLPs.

Further disclosed is a method for the manufacture of a vaccine, the method comprising a) combining HPV 16 VLPs, HPV 18 VLPs and an adjuvant to form a vaccine, and b) filling storage or delivery vessels with a human dose of the vaccine containing 60 µg of HPV 16 VLPs and 60 µg of HPV 18 VLPs.

### Brief Description of the Figures

Figure 1 shows geometric mean titres for anti-HPV-16 antibody titres in subjects receiving a 2 dose HPV vaccination, one month after the last dose of HPV vaccine, as described in Example 2.
Figure 2 shows geometric mean titres for anti-HPV-18 antibody titres in subjects receiving a 2 dose HPV vaccination, one month after the last dose of HPV vaccine, as described in Example 2.

### Detailed description

The invention describes for the first time a two dose HPV vaccine. Disclosed is a method for the prevention of human papillomavirus related disease or infection by administering a two dose HPV vaccine. The method comprises delivering to an individual in need thereof a vaccine comprising HPV 16 and HPV 18 virus like particles (VLPs) together with a pharmaceutically acceptable adjuvant, wherein the vaccine is delivered in two consecutive doses consisting of a first dose and a second dose.

The use of a two dose regimen compared to a three dose regimen offers the possibility of improving patient compliance and the possibility of HPV vaccination being more compatible with other adolescent vaccine schedules. Two visits to the physician rather than three also offers benefits to the healthcare systems.

Vaccine can be administered in two doses wherein each dose of the vaccine comprises HPV 16 VLPs and HPV 18 VLPs in a concentration of greater than 20 µg each. Each dose of the vaccine may contain, for example, 30 µg of each VLP, or 40 µg of each VLP, or 60 µg of each VLP, together with an adjuvant.

Vaccine can be administered in two doses wherein each dose of the vaccine comprises HPV 16 VLPs and HPV 18 VLPs in a concentration of 20 µg each.

Vaccine can be administered in two doses wherein each dose of the vaccine comprises HPV 16 VLPs and HPV 18 VLPs in a concentration of 40 µg and 20 µg respectively.

Administration of the vaccine can follow any 2-dose schedule, for example a 0, 1 month schedule, a 0, 2 month schedule, a 0, 3 month schedule, a 0, 4 month schedule, a 0, 5 month schedule or a 0, 6 month schedule. For example the second dose is administered between 2 weeks and 8 months after administration of the first dose, for example between 1 and 6 months after the first dose or between 3 and 8 months after the first dose. Thus the second dose may be administered for example one month or two months or three months or four months or five months or six months after the first dose.

The second dose of vaccine can be administered more than two months after the first dose, for example 3 or more months, or 4 or more months, or 5 or more months, or 6 or more months after the first dose, where in each case there can be an upper limit of 8 months after the first dose.

HPV 16 and HPV 18 VLPs can be employed, each in an amount greater than 20 µg per human dose, for example 30 µg per dose or greater than 30 µg per dose, for example 40 µg per dose or 60 µg per dose or 80 µg per dose. The amount of HPV 16 and 18 VLPs per dose can be the same or different. The amount of HPV 16 and 18 VLPs can be each independently in the range 25 to 85 µg per dose, 30 to 50 µg per dose, or suitably 35 to 45 µg per dose.

The term "vaccine" as used herein refers to a composition that comprises an immunogenic component capable of provoking an immune response in an individual, such as a human, wherein the composition optionally contains an adjuvant. A vaccine for HPV suitably elicits a protective immune response against incident infection, or persistent infection, or cytological abnormality such as ASCUS, CIN1, CIN2, CIN3, or cervical cancer caused by one or more HPV types.

By the term "human dose" is meant a dose which is in a volume suitable for human use. Generally this is a liquid between 0.3 and 1.5 ml in volume. In one embodiment, a human dose is 0.5 ml. In a further embodiment, a human dose is higher than 0.5 ml, for example 0.6, 0.7, 0.8, 0.9 or 1 ml. In a further embodiment, a human dose is between 1 ml and 1.5 ml.

VLPs from HPV types in addition to HPV 16 and HPV 18 can be employed. In particular, other VLPs from other HPV types that can be included in the vaccine, use and method include VLPs from one or more oncogenic HPV types such as HPV 31, 33, 35, 39, 45, 51, 56, 58, 59, 66 and 68. Other VLPs from other HPV types that can be employed include VLPs from non-oncogenic HPV types such as HPV 6 and HPV 11.

HPV 16 and HPV 18 only VLPs, can be employed.

HPV 16, HPV 18, HPV 6 and HPV 11 VLPs, either alone or in combination with VLPs of one or more other oncogenic HPV types, can be employed.

HPV 16, HPV 18, HPV 6 and HPV 11 only VLPs can be used and each dose of the vaccine comprises HPV 16, HPV 18, HPV 6 and HPV 11 VLPs in a concentration of 40 µg, 20 µg, 20 µg 40 µg respectively.

HPV VLPs and methods for the production of VLPs are well known in the art. VLPs typically are constructed from the HPV L1 and optionally L2 structural proteins of the virus. See for example WO9420137, US5985610, WO9611272, US6599508B1, US6361778B1, EP595935. Any suitable HPV VLP may be used, such as an L1-only VLP or a VLP comprising an L1 and L2 protein.

The VLPs can be composed of only L1 protein or immunogenic fragments thereof, or of both L1 or immunogenic fragments thereof and L2 or immunogenic fragments thereof.

In any of the embodiments described herein the HPV VLPs can comprise HPV L1 protein or an immunogenic fragment thereof. The VLPs can further comprise a peptide from another HPV protein.

The VLPs can be L1-only VLPs composed of L1 or an immunogenic fragment thereof.

Where an immunogenic fragment of L1 is used, then suitable immunogenic fragments of HPV L1 include truncations, deletions, substitution, or insertion mutants of L1. Such immunogenic fragments can be capable of raising an immune response, said immune response being capable of recognising an L1 protein such as L1 in the form of a virus particle or VLP, from the HPV type from which the L1 protein was derived.

Immunogenic L1 fragments that can be used include truncated L1 proteins. In one aspect the truncation removes a nuclear localisation signal and optionally also removes DNA binding patterns in the L1 C terminal region. In another aspect the truncation is a C terminal truncation. In a further aspect the C terminal truncation removes fewer than 50 amino acids, such as fewer than 40 amino acids. Where the L1 is from HPV 16 then in another aspect the C terminal truncation removes 34 amino acids from the carboxy terminus of the HPV 16 L1. Where the L1 is from HPV 18 then in a further aspect the C terminal truncation removes 35 amino acids from the carboxy terminus of the HPV 18 L1. Thus a truncated L1 protein can be truncated at the C terminal compared to the wild type L1, so as to remove the nuclear localisation signal and optionally also DNA binding patterns, for example by removal of fewer than 50 or fewer than 40 amino acids from the C terminal end of the protein. Examples of such truncated proteins for L1 from HPV 16 and 18 are given below as SEQ ID Nos: 1 and 2. Truncated L1 Proteins are also described in US 6,060,324, US 6,361,778, and US 6,599,508 incorporated herein by reference.

In one aspect the HPV 16 L1 amino acid sequence is the following sequence:

(SEQ ID NO: 1)
MSLWLPSEATVYLPPVPVSKVVSTDEYVARTNIYYHAGTSRLLAVGHPYFPIKKPNNNKI 60
LVPKVSGLQYRVFRIHLPDPNKFGFPDTSFYNPDTQRLVWACVGVEVGRGQPLGVGISGH 120
PLLNKLDDTENASAYAANAGVDNRECISMDYKQTQLCLIGCKPPIGEHWGKGSPCTNVAV 180
NPGDCPPLELINTVIQDGDMVDTGFGAMDFTTLQANKSEVPLDICTSICKYPDYIKMVSE 240
PYGDSLFFYLRREQMFVRHLFNRAGAVGENVPDDLYIKGSGSTANLASSNYFPTPSGSMV 300
TSDAQIFNKPYWLQRAQGHNNGICWGNQLFVTVVDTTRSTNMSLCAAISTSETTYKNTNF 360
KEYLRHGEEYDLQFIFQLCKITLTADVMTYIHSMNSTILEDWNFGLQPPPGGTLEDTYRF 420

VTSQAIACQKHTPPAPKEDPLKKYTFWEVNLKEKFSADLDQFPLGRKFLLQ 471

The HPV 16 L1 sequence can also be that disclosed in WO94/05792 or US 6,649,167, for example, suitably truncated. Suitable truncates are truncated at a position equivalent to that shown above, as assessed by sequence comparison, and using the criteria disclosed herein.

In one aspect the HPV 18 L1 amino acid sequence is the following sequence:

(SEQ ID NO: 2)

MALWRPSDNTVYLPPPSVARVVNTDDYVTRTSIFYHAGSSRLLTVGNPYFRVPAGGGNKQ 60

DIPKVSAYQYRVFRVQLPDPNKFGLPDNSIYNPETQRLVWACVGVEIGRGQPLGVGLSGH 120

PFYNKLDDTESSHAATSNVSEDVRDNVSVDYKQTQLCILGCAPAIGEHWAKGTACKSRPL 180

SQGDCPPLELKNTVLEDGDMVDTGYGAMDFSTLQDTKCE-VPLDICQSICKYPDYLQMSAD 240

PYGDSMFFCLRREQLFARHFWNRAGTMGDTVPPSLYIKGTGMRASPGSCVYSPSPSGSIV 300

TSDSQLFNKPYWLHKAQGHNNGVCWHNQLFVTVVDTTRSTNLTICASTQSPVPGQYDATK 360

FKQYSRHVEEYDLQFIFQLCTITLTADVMSYIHSMNSSILEDWNFGVPPPPTTSLVDTYR 420

FVQSVAITCQKDAAPAENKDPYDKLKFWNVDLKEKFSLDLDQYPLGRKFLVQ 472

An alternative HPV 18 L1 sequence is disclosed in WO96/29413, which can be suitably truncated. Suitable truncates are truncated at a position equivalent to that shown above, as assessed by sequence comparison, and using the criteria disclosed herein.

Other HPV 16 and HPV 18 L1 sequences are well known in the art and can be suitable for use in the present invention.

An HPV early antigen, for example an antigen selected from the group consisting of HPV E1, E2, E3, E4, E5, E6, E7, or E8, may be employed.

The combination and quantity of HPV VLPs and/or antigens may not significantly impact the immunogenicity of any one HPV VLP or antigen, in particular the HPV 16 and HPV 18 VLPs. There may be no biologically relevant interference between HPV VLPs and antigens used in combination, such that the use of a combination of VLPs and antigens from different HPV types is able to induce an appropriate immune response and offer effective protection against infection or disease caused by each HPV genotype represented in the vaccine.

The immune response against a given HPV type in the combination may be at least 50% of the immune response of that same HPV type when measured individually, or 100% or substantially 100%. For responses to the HPV 16 and HPV 18, the combined vaccine of the invention preferably stimulates an immune response which is at least 50% of that provided by a combined HPV 16 / HPV 18 vaccine. The immune response generated by the vaccine may be at a level in which the protective effect of each HPV type is still seen. The immune response can be measured, for example, by antibody responses, in either preclinical or human experiments. Measurement of antibody responses is well known in the art, and disclosed in (for example) WO03/077942.

VLPs can be made in any suitable cell substrate such as yeast cells or insect cells e.g. using a baculovirus system in insect cells, and techniques for preparation of VLPs are well known in the art, such as WO9913056, US 6416945B1, US 6261765B1 and US6245568, and references therein, the entire contents of which are hereby incorporated by reference.

VLPS can be made by disassembly and reassembly techniques. For example, McCarthy et al, 1998 "Quantitative Disassembly and Reassembly of Human Papillomavirus Type 11 Virus like Particles in Vitro" J. Virology 72(1):33-41, describes the disassembly and reassembly of recombinant L1 HPV 11 VLPs purified from insect cells in order to obtain a homogeneous preparation of VLPs. WO99/13056 and US6245568 also describe disassembly/reassembly processes for making HPV VLPs.

In one embodiment HPV VLPS are made as described WO99/13056 or US6245568.

Alternatively VLPs can be made by expressing the L1 protein or immunogenic fragment, extracting it from the production system or cell substrate and purifying the protein while it is predominantly in the form of L1 monomers or pentamers (capsomers), and then forming VLPs from the purified protein. In one embodiment, the extraction and/or purification step is carried out in the presence of a reducing agent such as β-mercaptoethanol (BME), to prevent VLP formation. In one embodiment, the process comprises the step of removing the reducing agent such as BME to allow VLPs to spontaneously form.

VLP formation can be assessed by standard techniques such as, for example, electron microscopy and dynamic laser light scattering.

Optionally the vaccine can also be formulated or co-administered with other, non-HPV antigens. Suitably these non-HPV antigens can provide protection against other diseases, such as sexually transmitted diseases such as herpes simplex virus. For example the vaccine may comprise gD or a truncate thereof from HSV. In this way the vaccine provides protection against both HPV and HSV.

The vaccine may be provided in a liquid vaccine formulation, although the vaccine can be lyophilised and reconstituted prior to administration.

An adjuvant or a mixture of adjuvants, may be employed in combination with the VLPs. The VLPs can be used in combination with aluminium, and can be adsorbed or partially adsorbed onto aluminium adjuvant. Other adjuvants which can be used are adjuvants which stimulate a Th1 type response such as lipopolysaccharides, for example a nontoxic derivative of lipid A, such as monophosphoryl lipid A or more particularly 3-*O*-desacyl-4'-monophoshoryl lipid A (3D- MPL). Suitably the adjuvant is an aluminium salt, preferably in combination with a lipopolysaccharide such as 3D-MPL.

In one embodiment the adjuvant is aluminium hydroxide, or the combination of aluminium hydroxide with 3D-MPL.

When VLPs are adsorbed on to aluminium containing adjuvants, the VLPs can be adsorbed to the aluminium adjuvant prior to mixing of the VLPs to form the final vaccine product.

3D-MPL is sold under the name MPL by GlaxoSmithKline Biologicals N.A. and is referred to throughout the document as MPL or 3D-MPL. See, for example, US Patent Nos. 4,436,727; 4,877,611; 4,866,034 and 4,912,094. 3D-MPL primarily promotes CD4+ T cell responses with an IFN-g (Th1) phenotype. 3D-MPL can be produced according to the methods disclosed in GB 2 220 211 A or US 4,912,094. Chemically it is a mixture of 3-deacylated monophosphoryl lipid A with 3, 4, 5 or 6 acylated chains. In one embodiment small particle 3D-MPL is used. Small particle 3D-MPL has a particle size such that it may be sterile-filtered through a 0.22µm filter. Such preparations are described in WO 94/21292.

The amount of 3D-MPL in each dose of vaccine is suitably able to enhance an immune response to an antigen in a human. In particular a suitable 3D-MPL amount is that which improves the immunological potential of the composition compared to the unadjuvanted composition, or compared to the composition adjuvanted with another MPL amount, whilst being acceptable from a reactogenicity profile.

The amount of 3D-MPL in each dose of vaccine can be for example between 1-200 µg, or between 10-100 µg, or between 20-80 µg for example 25 µg per dose, or between 40-60 µg for example 50 µg per dose.

The bacterial lipopolysaccharide derived adjuvants to be formulated in the compositions described herein can be purified and processed from bacterial sources, or alternatively they can be synthetic. For example, purified monophosphoryl lipid A is described in Ribi et al 1986 (supra), and 3-O-desacylated monophosphoryl or diphosphoryl lipid A derived from *Salmonella sp.* is described in GB 2220211 and US 4912094. Other purified and synthetic lipopolysaccharides have been described (Hilgers et al., 1986, Int.Arch.Allergy.Immunol., 79(4):392-6; Hilgers et al., 1987, Immunology, 60(1):141-6; and EP 0 549 074 B1).

The vaccine can also comprise aluminium or an aluminium compound as a stabiliser.

The vaccine described herein can be administered by any of a variety of routes such as oral, topical, subcutaneous, musosal (typically intravaginal), intraveneous, intramuscular, intranasal, sublingual,intradermal and via suppository. Intramuscular and intradermal delivery are preferred.

The vaccine described herein can be tested using standard techniques, for example in standard preclinical models, to confirm that the vaccine is immunogenic.

In respect of the disclosure made, the vaccine may be used for the vaccination of adolescent girls aged from 9 and older e.g. 10-15, such as 10-13 years. However, older girls above 15 years old and adult women can also be vaccinated. Similarly the vaccine can be administered to younger age groups such as 2-12 year olds. However, the vaccine can also be administered to women following an abnormal pap smear or after surgery following removal of a lesion caused by HPV, or who are seronegative and DNA negative for HPV cancer types.

In the disclosure made herein, the vaccines and methods are for use in females in one or more of the following age brackets: 9 to 25 years of age, 10 to 25 years of age, 9 to 19 years of age, 10 to 19 years of age, 9 to 14 years of age, 10 to 14 years of age, 15 to 19 years of age, 20 to 25 years of age, 14 years of age or below, 19 years of age or below, 25 years of age or below.

The vaccines and methods described herein can be used in men or boys.

The following examples are to be understood as illustrative of the claimed invention insofar as such relate to the claimed subject matter. Insofar as not relating to the claimed subject matter, the examples are provided for information purposes only.

### EXAMPLES

### Example 1 - Preparation of HPV 16/18 L1 VLPs

Production of HPV 16, HPV 18, HPV 33 and HPV 58 L1 VLPs was carried out using standard protocols - for example, see WO9913056.

The HPV L1 gene encoding each of the L1 proteins was deleted at its 3'-end prior to its cloning in a Baculovirus expression vector to remove the nuclear localization and DNA binding patterns initially present at the C-terminus of each of the L1 proteins. Standard genetic manipulations resulted in the cloning of C-terminally truncated genes (C-terminal end deletions of 34 and 35 amino acids, respectively for HPV 16 and 18). Amino acid sequences of the HPV 16 and 18 L1 truncates as used herein are given in the description (as SEQ ID NOs: 1 and 2 respectively).

HPV 16 and 18 truncated L1 proteins were expressed in *Trichoplusia ni* (High Five™) cells (at a density of ∼ 2 000 000 cells/ml) infected with recombinant Baculovirus (MOI of 0.5) encoding the HPV 16/18 L1 gene of interest. Cells were harvested approximately 72 to 96 hours post infection.

### Cell harvest / antigen extraction

The antigen (L1-16/18) was extracted from Hi5 cells in a three step process of concentration, extraction, clarification. The concentration step removes up to 90% of the culture medium, and was performed by centrifugation. The extraction step was performed with a hypotonic buffer (Tris 20mM, pH 8.5). A volume equal to the culture volume was used to perform the extraction. A contact time of minimum half an hour under smooth agitation was used. The clarification was performed by tangential flow filtration.

### Purification

The purification process was carried out at room temperature. β-mercaptoethanol (BME) (4% w/w) was added to the extract in order to prevent VLP formation.

All buffers used were filtered on 0.22µm filters. Prior to each purification run, gel matrixes are sanitised and equilibrated with appropriate buffer before sample loading.

Purification regimes are given for the separate purification of L1 from HPV 16 and HPV 18. These schemes are broadly similar, and involve the steps of:
Anion exchange chromatography (Di methyl amino ethyl - DMAE),
Anion exchange chromatography (tri methyl amino ethyl - TMAE),
Hydroxyapatite chromatography,
Nanometric filtration (Planova),
Ultrafiltration
Hydrophobic interaction chromatography (using Octyl Sepharose) for HPV 18 or
Anion exchange chromatography (DEAE) for HPV 16; and
Sterile filtration.

### Purification of L1-18 antigen

### Anion exchange chromatography DMAE

The clarified extract (protein at a concentration of ∼ 1 mg/ml, with the L1 protein at ∼ 150 µg/ml) was applied to an anion exchange column (Di Methyl Amino Ethyl). Elution was performed with (Tris 20mM |NaCl 200mM| 4% β-mercaptoethanol BME) buffer, pH 7.9 ± 0.2 . The antigen was eluted in approximately 5 column volumes and the elution profile was monitored at 280 nm.

### Anion exchange chromatography TMAE

The eluate of the first step was diluted with 1 volume of H₂O/BME 4%. The diluted eluate was then applied to a second anion exchange column (Tri Methyl Amino Ethyl).

Elution was performed with (20mM Tris |NaCl 200mM| 4%BME) buffer, pH 7.9 ± 0.2. The antigen was eluted in approximately 4 column volumes and the elution profile was monitored at 280 nm.

### Hydroxyapatite chromatography

The eluate of the TMAE step was applied to a hydroxyapatite (HA) column. After sample application, the gel was eluted with approximately 2.5 column volumes of (NaH₂PO₄ 100mM| |NaCl 30mM| 4%BME) buffer, pH 6.0 ± 0.2.

### Nanometric filtration (Planova)

The HA eluate was diluted in order to reach the following conditions: (NaH₂PO₄ 25mM |NaCl 10mM| 4%BME) buffer, pH 7.5 ± 0.2.

Then it was filtered successively on a 0.2 µm prefilter and on a Planova 15N filter of 0.12 m². The filtration was performed at constant pressure 200 mbar ± 20 mbar.

### Ultrafiltration

The ultrafiltration was performed with a tangential flow ultrafiltration system equipped with polyethersulfone membranes (Centramate cassette 0.1 m², 100kD).

The Planova eluate was treated to reach the following conditions: (NaH₂PO₄ 100mM |NaCl 30mM| 4%BME), pH 6.0 ± 0.2 ; then it was loaded in the system, concentrated 5 fold and dia-filtrated with continuous injection of ∼10 starting volumes of (NaH₂PO₄ 20mM |NaCl 500mM) buffer, pH 6.0 ± 0.2.

### Hydrophobic interaction chromatography (Octyl Sepharose)

The ultrafiltration permeate was applied to an Octyl Sepharose column.

This chromatography step was run in the negative mode with approximately 5 column volumes of (Na₃PO₄ 20mM |NaCl 500mM) buffer, pH 6.0 ± 0.2 .

### Sterile filtration

The purified L1-18 antigen solution was sterilised by filtration on a 0.22 µm membrane.

### Purification of L1-16 antigen

### Anion exchange chromatography DMAE

The clarified extract was applied to an anion exchange column (Di Methyl Amino Ethyl).

Elution was performed with (Tris 20mM |NaCl 180mM| 4%BME) buffer, pH 7.9 ± 0.2. The antigen was eluted in approximately 4 column volumes and the elution profile was monitored at 280 nm.

### Anion exchange chromatography TMAE

The eluate of the first step was diluted with 1 volume of H₂O/BME 4%. The diluted eluate was then applied to a second anion exchange column (Tri Methyl Amino Ethyl).

Elution was performed with (20mM Tris |Nacl 180mM| 4%BME) buffer, pH 7.9 ± 0.2. The antigen was eluted in approximately 5 column volumes and the elution profile was monitored at 280 nm.

### Hydroxyapatite chromatography (HA)

The eluate of the TMAE step was applied to a HA column.

After sample application, the gel was eluted with approximately 3 column volumes of (NaH₂PO₄ 100mM| NaCl 30mM| 4%BME) buffer, pH 6.0 ± 0.2.

### Nanometric filtration (Planova)

The HA eluate was diluted in order to reach the following conditions: (NaH₂PO₄ 25mM | NaCl 10mM| 4%BME) buffer, pH 7.5 ± 0.2.

Then it was filtered successively on a 0.2 µm prefilter and on a Planova 15N filter of 0.12 m². The filtration was performed at constant pressure 200 mbar ± 20 mbar.

### Ultrafiltration

The ultrafiltration was performed with a tangential flow ultrafiltration system equipped with polyethersulfone membranes (Centramate cassette 0.1 m², 100kD).

The Planova eluate was treated to reach the following conditions: (NaH₂PO₄ 100mM |NaCl 30mM| 4%BME), pH 6.0 ± 0.2 ; then it was loaded in the system, concentrated 5 fold and dia-filtrated with continuous injection of ∼10 starting volumes of (NaH₂PO₄ 20mM| NaCl 500mM) buffer, pH 6.0 ± 0.2 .

### Anion exchange chromatography DEAE

The ultrafiltration eluate was adjusted to the conductivity of the equilibrium buffer, (Na₃PO₄ 20mM| NaCl 250mM), pH 6.0 ± 0.2 and applied on an anion exchange column (Di Ethyl Amino Ethyl).

Elution was performed with (NaH₂PO₄ 20mM| NaCl 500mM) buffer, pH 6.0 ± 0.2. The antigen was eluted in approximately 3 column volumes and the elution profile was monitored at 280 nm.

### Sterile filtration

The purified L1-16 antigen solution was sterilised by filtration on a 0.22 µm membrane.

### Example 2 - A phase I/II, partially blind, randomized, multicenter, age-stratified, dose-range study in healthy females aged 9 - 25 years to assess the safety and immunogenicity of HPV-16/18 L1 VLP AS04 vaccine administered intramuscularly according to a 2-dose schedule (0,2-month or 0,6-month) when compared to a standard 3- dose schedule of GlaxoSmithKline Biologicals' HPV-16/18 L1 VLP AS04 vaccine.

### Primary Objectives (immunogenicity):

### Immunogenicity

- To evaluate the immunogenicity of the HPV-16/18 L1 VLP AS04 vaccine one month after the last dose when administered at different dosages (20 or 40 µg of each HPV antigen) and on different schedules (0,2- or 0,6-months) compared with the standard HPV-16/18 L1 VLP AS04 vaccine administered on a 3-dose schedule (0,1,6-months).

### Secondary Objectives (immunogenicity):

### The three following objectives were assessed:

### • The first secondary objective for immunogenicity was:

To demonstrate the non-inferiority of the antibody response to the 2-dose schedule of the HPV-16/18 L1 VLP AS04 vaccine in the 9 - 14 year age stratum when administered at different dosages (20 or 40 µg of each HPV antigen) and on different schedules (0,2- and 0,6-months) as compared to the standard 3-dose schedule in subjects 15 - 25 years of age (the age group in which efficacy has been demonstrated), one month after the last dose of vaccine.

### Criteria to be used for non-inferiority:

*Non-inferiority was demonstrated if the upper limit of the 95% confidence interval (CI) for the geometric mean titer (GMT) ratio between the standard 3-dose schedule of HPV 16*/*18 L 1 VLP AS04 vaccine in subjects 15 - 25 years of age over the 2-dose schedules in the 9 - 14 year age stratum was below 2.*

### • the next secondary objective to be evaluated:

To demonstrate the non-inferiority of the antibody response to the 2-dose schedule of the HPV-16/18 L1 VLP AS04 vaccine in the 15 - 19 year age stratum when administered at different dosages (20 or 40 µg of each HPV antigen) and on different schedules (0,2- or 0,6-months) as compared to the standard 3-dose schedule in subjects 15 - 25 years of age, one month after the last dose of vaccine.

### Criteria to be used for non-inferiority:

*Non-inferiority was demonstrated if the upper limit of the 95% Cl for the GMT ratio between the standard 3-dose schedule of HPV- 16*/*18 L 1 VLP AS04 vaccine in subjects 15 - 25 years of age over the 2-dose schedules in the 15 - 19 year age stratum was below 2.*

### • third secondary objective to be evaluated:

To demonstrate the non-inferiority of the antibody response to the 2-dose schedule of the HPV-16/18 L1 VLP AS04 vaccine in the 20 - 25 year age stratum when administered at different dosages (20 or 40 µg of each HPV antigen) and on different schedules (0,2- or 0,6-months) as compared to the standard 3-dose schedule in subjects 15 - 25 years of age, one month after the last dose of vaccine.

### Criteria to be used for non-inferiority:

*Non-inferiority was demonstrated if the upper limit of the 95% CI for the GMT ratio between the standard 3-dose schedule of HPV-16*/*18 L1 VLP AS04 vaccine in subjects 15 - 25 years of age over the 2-dose schedules in the 20 - 25 year age stratum was below 2.*

### • If any of the above secondary objectives for immunogenicity were not demonstrated, the following objective was to be evaluated:

To examine pair wise comparisons of the antibody response between each 2-dose schedule group and the standard 3-dose schedule, one month after the last dose of vaccine within each age stratum.

To evaluate the antibody response to all dose schedules and dosages of the HPV-16/18 L1 VLP AS04 vaccine in each age stratum during the extended follow-up period (at Month 12, Month 18 and Month 24).

### Study Design

- Experimental design: a phase I/II, partially blind, controlled, randomized, age-stratified trial with four parallel groups. Each group was stratified into three age strata: 9 - 14, 15 - 19 and 20 - 25 years of age.
- Treatment groups: Four groups received the HPV-16/18 L1 VLP AS04 at different dosages (20 µg or 40 µg of each HPV antigen) and on different schedules (0,2-month, 0,6-month or 0,1,6-month schedules):

**Table 1**

| Group | Abbreviation | HPV-16/18 dosages (µg/µg) | Schedules | Age strata (years) |
|---|---|---|---|---|
| **40/40 M0,2** | **V40_02** | **40/40** | **0,2-month** | **9 - 14** |
| | | | | **15 - 19** |
| | | | | **20 - 25** |
| **40/40 M0,6** | **V40_06** | **40/40** | **0,6-month** | **9 - 14** |
| | | | | **15 - 19** |
| | | | | **20 - 25** |
| **20/20 M0,6** | **V20_06** | **20/20** | **0,6-month** | **9 - 14** |
| | | | | **15 - 19** |
| | | | | **20 - 25** |
| **20/20 M0,1,6** | **HPV** | **20/20** | **0,1,6-month** | **9 - 14** |
| | | | | **15 - 19** |
| | | | | **20 - 25** |

- The trial was observer-blind within the 2-dose schedule groups (40/40 M0,2; 40/40 M0,6 and 20/20 M0,6) and open in the standard 3-dose schedule group (20/20 M0,1,6).
- Vaccination schedules:
   - Group 40/40 M0,2: two doses of HPV-16/18 L1 VLP (40 µg/40 µg) AS04 vaccine administered at Months 0 and 2*.
   - Groups 40/40 M0,6 and 20/20 M0,6: two doses of HPV-16/18 L1 VLP (40 µg / 40 µg or 20 µg / 20 µg) AS04 vaccine administered at Months 0 and 6*.
      *for blinding within these groups, a placebo [Al(OH)₃] was to be administered at Month 6 (Group 40/40 M0,2) or at Month 2 (Groups 40/40 M0,6 and 20/20 M0,6).
   - Group 20/20 M0,1,6: three doses of HPV-16/18 L1 VLP (20 µg/20 µg) AS04 vaccine administered at Months 0, 1 and 6.
- Study visits: depending on the group to which the subject was assigned, there were 7 (for the 3-dose schedule group) or 8 (for the 2-dose schedule groups) visits per subject.
   - For the 2-dose schedule groups: blood samples were drawn at Visit 1 (Month 0), Visit 3 (Month 3) and Visit 5 (Month 7), and will be drawn at Visit 6 (Month 12), Visit 7 (Month 18) and Visit 8 (Month 24).
   - For the 3-dose schedule group: blood samples were drawn at Visit 1 (Month 0) and Visit 4 (Month 7), and will be drawn at Visit 5 (Month 12), Visit 6 (Month 18) and Visit 7 (Month 24).
- Control: GSK Biologicals' HPV-16/18 L1 VLP AS04 vaccine administered at Month 0, Month 1 and Month 6 in subjects aged 15 - 25 years (pooling of 15-19 and 20-25 years age strata).

### Number of subjects:

*Planned:* Approximately 960 subjects: Approximately 240 subjects per group and approximately 80 subjects per age stratum.

*Enrolled:* 961 subjects were enrolled in the study.

*Completed:* 922 subjects (231, 228, 229 and 234 subjects in the 40/40 M0,2, 40/40 M0,6, 20/20 M0,6 and standard HPV-16/18 L1 VLP AS04 vaccine groups, respectively), completed the active phase of the study.

*Safety:* Total Vaccinated cohort: 960 subjects (240, 241, 240 and 239 subjects in the 40/40 M0,2, 40/40 M0,6, 20/20 M0,6 and standard HPV-16/18 L1 VLP AS04 vaccine groups, respectively).

*Immunogenicity:* ATP cohort: 843 subjects (224, 206, 205 and 208 subjects in the 40/40 M0,2, 40/40 M0,6, 20/20 M0,6 and standard HPV-16/18 L1 VLP AS04 vaccine groups, respectively).

### Diagnosis and criteria for inclusion:

- Subjects who the investigator believed that they and/or their parents could and would comply with the requirements of the protocol.
- A female subject between, and including, 9 and 25 years of age at the time of the first vaccination.
- Written informed consent/assent obtained from the subject prior to enrolment.
- Healthy subjects as established by medical history and history-oriented clinical examination.

Subjects were to be of non-childbearing potential.

### Study vaccine:

### Vaccination schedule /site:

- Vaccines were administered intramuscularly (IM)

### Vaccine composition /dose:

- Group 40/40 M0,2 and M0,6: Each dose (0.5 mL) contained 40 µg HPV-16 L1 protein, 40 µg HPV-18 L1 protein, 50 µg 3-O-desacyl-4'-monophosphoryl lipid A (MPL), 500 µg aluminum hydroxide [Al(OH)₃], 180 mM NaCl, 8 mM NaH₂PO₄.2H₂O and q.s. ad 0.5 mL water for injection.
- Groups 20/20 M0,6: Each dose (0.5 mL) contained 20 µg HPV-16 L1 protein, 20 µg HPV-18 L1 protein, 50 µg MPL, 500 µg Al(OH)₃, 150 mM NaCl, 8 mM NaH₂PO₄.2H₂O, q.s. ad 0.5 mL water for injection.
- Placebo: Each dose (0.5 mL) contained 500 µg Al(OH)₃, 150 mM NaCl, 8 mM NaH₂PO₄.2H₂O, q.s ad 0.5 mL water for injection.

### Reference vaccine:

### Vaccination schedule /site:

- Group 20/20 M0,1,6: three doses of HPV-16/18 L1 VLP (20µg/20µg) AS04 vaccine administered at Months 0, 1 and 6.

### Vaccine composition /dose:

- Group 20/20 M0,1,6: Each dose (0.5 mL) of HPV-16/18 L1 VLP AS04 vaccine contained 20 µg HPV-16 L1 VLP, 20 µg HPV-18 L1 VLP, 50 µg MPL, 500 µg Al(OH)₃, 4.4 mg NaCl, 0.624 mg NaH₂PO₄ 2H₂O and water for injection.

### Criteria for evaluation:

### Co-Primary endpoints:

Immunogenicity: HPV-16 and HPV-18 antibody titers (by ELISA) assessed one month after the last dose of vaccine when administered at different dosages (20 or 40 µg of each HPV type) and on different schedules (0,2- or 0,6 or 0,1,6-months).

Safety: Occurrence, intensity and causal relationship to vaccination of solicited local and general symptoms within 7 days (Days 0 - 6) after each and any vaccination.

### Secondary endpoints:

### Immunogenicity

- HPV-16 and HPV-18 antibody titers (by ELISA) assessed one month after the last dose of vaccine or placebo (Month 7) in all study groups and in all age strata.
- HPV-16 and HPV-18 antibody titers (by ELISA) assessed one month after the second dose of vaccine or placebo in the 2-dose schedule groups (Month 3).
- HPV-16 and HPV-18 antibody titers (by ELISA) and seroconversion status assessed during the extended follow-up period (at Month 12, Month 18 and Month 24).

### Analysis of immunogenicity:

The primary analysis of immunogenicity was based on the ATP cohort.

For each group at each time point that a blood sample result was available:
- Seropositivity rates for anti-HPV-16 and anti-HPV-18 (with exact 95% CI) were calculated per pre-vaccination status;
- Anti-HPV-16 and anti-HPV-18 GMTs with 95% CI and range of antibody titers were tabulated per pre-vaccination status;
- The distribution of antibody titers for anti-HPV-16 and anti-HPV-18 one month after the last dose of active vaccine were displayed using reverse cumulative distribution curves for the sub-cohort of initially seronegative subjects.

### Analysis of the primary objective:

A two-way ANOVA model was applied using titers in logarithm 10 as response variable for anti-HPV-16 and anti-HPV-18 separately. The model contained age, group and group-by-age interactions as fixed factors. The interaction term (group-by-age) was tested at 10%. If the group-by-age interaction term was not significant at 10% further estimations were to be drawn across all age strata. Dunnett's multiple comparisons were to be performed. If the interaction was significant at 10%, pair wise comparisons were to be made between each 2-dose schedule group and the 3-dose standard schedule group by age strata.

A one way ANOVA model was to be applied using titers in logarithm 10 as response variable for anti-HPV-16 and anti-HPV-18 separately by age strata. The model contained group as fixed factors and Dunnett's multiple comparisons were to be performed.

### Analyses of secondary objectives:

The following objectives were assessed sequentially:
The non-inferiority of the antibody response to the 2-dose schedule of the HPV-16/18 L1 VLP AS04 vaccine in the 9 - 14 years of age stratum when administered at different dosages (20 or 40 µg of each HPV antigen) and on different schedules (0,2- and 0,6-months) as compared to the standard 3-dose schedule in subjects 15 - 25 years of age, one month after the last dose of vaccine, were to be demonstrated, if the upper limit of the 95% CI for the GMT ratio between the standard 3-dose schedule of HPV-16/18 L1 VLP AS04 vaccine in subjects 15 - 25 years of age over the 2-dose schedules in the 9 - 14 year age stratum was below 2.

The second secondary objective, i.e. the non-inferiority of the antibody response to the 2-dose schedule of the HPV-16/18 L1 VLP AS04 vaccine in the 15- 19 year age stratum when administered at different dosages (20 or 40 µg of each HPV antigen) and on different schedules (0,2- or 0,6-months) as compared to the standard 3-dose schedule in subjects 15 - 25 years of age, one month after the last dose of vaccine, was to be demonstrated, if the upper limit of the 95% CI for the GMT ratio between the standard 3-dose schedule of HPV-16/18 L1 VLP AS04 vaccine in subjects 15 - 25 years of age over the 2-dose schedules in the 15 - 19 year age stratum was below 2.

The third secondary objective, i.e. the non-inferiority of the antibody response to the 2-dose schedule of the HPV-16/18 L1 VLP AS04 vaccine in the 20 - 25 year age stratum when administered at different dosages (20 or 40 µg of each HPV antigen) and on different schedules (0,2- or 0,6-months) as compared to the standard 3-dose schedule in subjects 15 - 25 years of age, one month after the last dose of vaccine, was to be demonstrated, if the upper limit of the 95% CI for the GMT ratio between the standard 3-dose schedule of HPV-16/18 L1 VLP AS04 vaccine in subjects 15 - 25 years of age over the 2-dose schedules in the 20 - 25 year age stratum was below 2.

If any of the above secondary objectives for immunogenicity were not demonstrated, pair wise comparisons of the antibody response between each 2-dose schedule group and the standard 3-dose schedule, one month after the last dose of vaccine within each age stratum were to be examined by using the Dunnett's method.

### Results

### Immunogenicity

The primary analysis of immunogenicity was performed on the ATP (according to protocol) cohort. A second analysis was performed on the Total Vaccinated cohort to supplement the ATP analysis.

### According-to-protocol analysis

Overall, 730 (86.6%) and 734 (87.1 %) subjects were seronegative at baseline for HPV-16 and HPV-18, respectively.

Seropositivity rates and GMTs for anti-HPV-16 antibody titers by serostatus at baseline and by group can be found in Table 2. Age stratified data are presented in Table 3. All subjects in all groups were seropositive one month after vaccination course (at Month 3 [one month post-dose II in the 40/40 M0,2 group] and Month 7 [one month post-dose II in the 40/40 M0,6 and 20/20 M0,6 groups and one month post-dose III in the standard HPV-16/18 L1 VLP AS04 vaccine group]). All subjects were also seropositive at Month 3, one month post-dose I in the 40/40 M0,6 and 20/20 M0,6 groups. Higher titers were measured for anti-HPV-16 in initially seronegative subjects at Month 7 and in initially seropositive subjects at Month 3, in the 20/20 M0,6 and 40/40 M0,6 groups.

**Table 2. Seropositivity rates and geometric mean titers (GMT) for anti-HPV-16 antibody titers bv group (ATP cohort for immunogenicity)**

| | | | | | **>= 8 ELU/ML** | | | | **GMT** | | | | |
|---|---|---|---|---|---|---|---|---|---|---|---|---|---|
| | | | | | | | **95% CI** | | | **95% CI** | | | |
| **Antibody** | **Group** | **Pre-vacc status** | **Timing** | **N** | **n** | **%** | **LL** | **UL** | **value** | **LL** | **UL** | **Min** | **Max** |
| HPV-16 | V40_02 | Total | PRE | 224 | 23 | 10.3 | 6.6 | 15.0 | 5.1 | 4.6 | 5.6 | <8.0 | 413.0 |
| | | | M3 | 224 | 224 | 100 | 98.4 | 100 | 5844.6 | 5259.6 | 6494.7 | 233.0 | 45534.0 |
| | V40_06 | Total | PRE | 204 | 31 | 15.2 | 10.6 | 20.9 | 6.0 | 5.1 | 7.1 | <8.0 | 1006.0 |
| | | | M7 | 204 | 204 | 100 | 98.2 | 100 | 10500.9 | 9356.9 | 11784.8 | 1211.0 | 57135.0 |
| | V20_06 | Total | PRE | 204 | 26 | 12.7 | 8.5 | 18.1 | 5.5 | 4.8 | 6.2 | <8.0 | 1259.0 |
| | | | M7 | 204 | 204 | 100 | 98.2 | 100 | 7741.6 | 6868.2 | 8726.1 | 603.0 | 47872.0 |
| | HPV | Total | PRE | 208 | 30 | 14.4 | 9.9 | 19.9 | 5.6 | 4.9 | 6.4 | <8.0 | 745.0 |
| | | | M7 | 208 | 208 | 100 | 98.2 | 100 | 13045.3 | 11211.4 | 15179.2 | 154.0 | 149951.0 |

| | | | | | | | | | | | | | |
|---|---|---|---|---|---|---|---|---|---|---|---|---|---|
| V40_02 = HPV-16/18(40,40) AS04 0,2 m V40_06 = HPV-16/18(40,40) AS04 0,6 m V20_06 = HPV-16/18(20,20) AS04 0,6 m HPV = HPV-16/18(20,20) AS04 0,1,6 m GMT = geometric mean antibody titer calculated on all subjects N = number of subjects with pre-vaccination results available n/% = number/percentage of subjects with titer within the specified range 95% CI = 95% confidence interval; LL = Lower Limit, UL = Upper Limit MIN/MAX = Minimum/Maximum PRE = Pre-vaccination | | | | | | | | | | | | | |

**Table 3 Seropositivity rates and geometric mean titers (GMT) for anti-HPV-16 antibody titers by age stratum, and by group (ATP cohort for immunogenicity)**

| | | | | | | **>=8 ELU/ML** | | | | **GMT** | | | | |
|---|---|---|---|---|---|---|---|---|---|---|---|---|---|---|
| | | | | | | | | **95% CI** | | | **95% CI** | | | |
| **Antibody** | **Group** | **Sub-group** | **Pre-vacc status** | **Timing** | **N** | **n** | **%** | **LL** | **UL** | **value** | **LL** | **UL** | **Min** | **Max** |
| HPV-16 | V40_02 | 9-14 | Total | PRE | 77 | 2 | 2.6 | 0.3 | 9.1 | 4.2 | 3.9 | 4.4 | <8.0 | 22.0 |
| | | | | M3 | 77 | 77 | 100 | 95.3 | 100 | 7391.6 | 6223.4 | 8779.1 | 966.0 | 45534.0 |
| | | 15-19 | Total | PRE | 73 | 3 | 4.1 | 0.9 | 11.5 | 4.3 | 4.0 | 4.7 | <8.0 | 36.0 |
| | | | | M3 | 73 | 73 | 100 | 95.1 | 100 | 5254.5 | 4358.2 | 6335.1 | 233.0 | 32270.0 |
| | | 20-25 | Total | PRE | 74 | 18 | 24.3 | 15.1 | 35.7 | 7.4 | 5.5 | 9.8 | <8.0 | 413.0 |
| | | | | M3 | 74 | 74 | 100 | 95.1 | 100 | 5084.4 | 4224.7 | 6118.9 | 668.0 | 22930.0 |
| | V40_06 | 9-14 | Total | PRE | 62 | 1 | 1.6 | 0.0 | 8.7 | 4.2 | 3.8 | 4.5 | <8.0 | 47.0 |
| | | | | M7 | 62 | 62 | 100 | 94.2 | 100 | 15028.4 | 12611.3 | 17908.6 | 2713.0 | 57135.0 |
| | | 15-19 | Total | PRE | 74 | 8 | 10.8 | 4.8 | 20.2 | 5.3 | 4.2 | 6.8 | <8.0 | 900.0 |
| | | | | M7 | 74 | 74 | 100 | 95.1 | 100 | 10818.7 | 8979.8 | 13034.2 | 1425.0 | 42798.0 |
| | | 20-25 | Total | PRE | 68 | 22 | 32.4 | 21.5 | 44.8 | 9.6 | 6.6 | 14.0 | <8.0 | 1006.0 |
| | | | | M7 | 68 | 68 | 100 | 94.7 | 100 | 7331.4 | 5965.2 | 9010.4 | 1211.0 | 48115.0 |
| | V20_06 | 9-14 | Total | PRE | 69 | 4 | 5.8 | 1.6 | 14.2 | 4.3 | 4.0 | 4.7 | <8.0 | 33.0 |
| | | | | M7 | 69 | 69 | 100 | 94.8 | 100 | 11058.6 | 9273.8 | 13186.7 | 2687.0 | 45919.0 |
| | | 15-19 | Total | PRE | 70 | 8 | 11.4 | 5.1 | 21.3 | 5.3 | 4.2 | 6.6 | <8.0 | 1259.0 |
| | | | | M7 | 70 | 70 | 100 | 94.9 | 100 | 7869.6 | 6488.9 | 9543.9 | 1290.0 | 47872.0 |
| | | 20-25 | Total | PRE | 65 | 14 | 21.5 | 12.3 | 33.5 | 7.4 | 5.4 | 10.1 | <8.0 | 337.0 |
| | | | | M7 | 65 | 65 | 100 | 94.5 | 100 | 5209.2 | 4166.5 | 6512.7 | 603.0 | 26064.0 |
| | HPV | 9-14 | Total | PRE | 75 | 8 | 10.7 | 4.7 | 19.9 | 4.6 | 4.2 | 5.0 | <8.0 | 26.0 |
| | | | | M7 | 75 | 75 | 100 | 95.2 | 100 | 22066.3 | 18140.7 | 26841.2 | 3932.0 | 149951.0 |
| | | 15-19 | Total | PRE | 66 | 6 | 9.1 | 3.4 | 18.7 | 5.1 | 4.1 | 6.3 | <8.0 | 742.0 |
| | | | | M7 | 66 | 66 | 100 | 94.6 | 100 | 12817.4 | 9723.2 | 16896.2 | 423.0 | 148276.0 |
| | | 20-25 | Total | PRE | 67 | 16 | 23.9 | 14.3 | 35.9 | 7.7 | 5.5 | 10.9 | <8.0 | 745.0 |
| | | | | M7 | 67 | 67 | 100 | 94.6 | 100 | 7370.0 | 5673.6 | 9573.6 | 154.0 | 125818.0 |

| | | | | | | | | | | | | | | |
|---|---|---|---|---|---|---|---|---|---|---|---|---|---|---|
| V40_02 = HPV-16/18(40,40) AS04 0,2 m V40_06 = HPV-16/18(40,40) AS04 0,6 m V20_06 = HPV-16/18(20,20) AS04 0,6 m HPV = HPV-16/18(20,20) AS04 0,1,6 m 9-14 = 9 to 14 years 15-19 =15 to 19 years 20-25 = 20 to 25 years GMT = geometric mean antibody titer calculated on all subjects N = number of subjects with pre-vaccination results available n/% = number/percentage of subjects with titer within the specified range 95% CI = 95% confidence interval; LL = Lower Limit, UL = Upper Limit MIN/MAX = Minimum/Maximum PRE = Pre-vaccination | | | | | | | | | | | | | | |

**Table 4 Seropositivity rates and geometric mean titers (GMT) for anti-HPV-18 antibody titers by group (ATP cohort for immunogenicity)**

| | | | | | **>= 7 ELU/ML** | | | | **GMT** | | | | |
|---|---|---|---|---|---|---|---|---|---|---|---|---|---|
| | | | | | | | **95% CI** | | | **95% CI** | | | |
| **Antibody** | **Group** | **Pre-vacc status** | **Timing** | **N** | **n** | **%** | **LL** | **UL** | **value** | **LL** | **UL** | **Min** | **Max** |
| HPV-18 | V40_02 | Total | PRE | 223 | 31 | 13.9 | 9.6 | 19.1 | 4.5 | 4.1 | 5.0 | <7.0 | 510.0 |
| | | | M3 | 223 | 223 | 100 | 98.4 | 100 | 3543.2 | 3126.6 | 4015.3 | 122.0 | 33321.0 |
| | V40_06 | Total | PRE | 206 | 22 | 10.7 | 6.8 | 15.7 | 4.3 | 3.9 | 4.8 | <7.0 | 387.0 |
| | | | M7 | 206 | 206 | 100 | 98.2 | 100 | 5997.5 | 5310.9 | 6772.8 | 412.0 | 91976.0 |
| | V20_06 | Total | PRE | 204 | 28 | 13.7 | 9.3 | 19.2 | 4.4 | 4.0 | 4.9 | <7.0 | 141.0 |
| | | | M7 | 204 | 204 | 100 | 98.2 | 100 | 4811.4 | 4282.7 | 5405.3 | 163.0 | 36047.0 |
| | HPV | Total | PRE | 208 | 26 | 12.5 | 8.3 | 17.8 | 4.3 | 4.0 | 4.7 | <7.0 | 161.0 |
| | | | M7 | 208 | 208 | 100 | 98.2 | 100 | 5087.1 | 4460.2 | 5802.1 | 391.0 | 84753.0 |

| | | | | | | | | | | | | | |
|---|---|---|---|---|---|---|---|---|---|---|---|---|---|
| V40_02 = HPV-16/18(40,40) AS04 0,2 m V40_06 = HPV-16/18(40,40) AS04 0,6 m V20_06 = HPV-16/18(20,20) AS04 0,6 m HPV = HPV-16/18(20,20) AS04 0,1,6 m N = number of subjects with pre-vaccination results available n/% = number/percentage of subjects with titer within the specified range 95% CI = 95% confidence interval; LL = Lower Limit, UL = Upper Limit MIN/MAX = Minimum/Maximum PRE = Pre-vaccination | | | | | | | | | | | | | |

**Table 5 Seropositivity rates and geometric mean titers (GMT) for anti-HPV-18 antibody titers by age stratum, and by group (ATP cohort for immunogenicity)**

| | | | | | | **>= 7 ELU/ML** | | | | **GMT** | | | | |
|---|---|---|---|---|---|---|---|---|---|---|---|---|---|---|
| | | | | | | | | **95% CI** | | | **95% CI** | | | |
| **Antibody** | **Group** | **Sub-group** | **Pre-vacc status** | **Timing** | **N** | **n** | **%** | **LL** | **UL** | **value** | **LL** | **UL** | **Min** | **Max** |
| HPV-18 | V40_02 | 9-14 | Total | PRE | 77 | 7 | 9.1 | 3.7 | 17.8 | 4.0 | 3.6 | 4.4 | <7.0 | 24.0 |
| | | | | M3 | 77 | 77 | 100 | 95.3 | 100 | 4990.8 | 4187.3 | 5948.6 | 562.0 | 33045.0 |
| | | 15-19 | Total | PRE | 73 | 5 | 6.8 | 2.3 | 15.3 | 4.0 | 3.5 | 4.5 | <7.0 | 114.0 |
| | | | | M3 | 73 | 73 | 100 | 95.1 | 100 | 3188.3 | 2550.3 | 3985.9 | 148.0 | 33321.0 |
| | | 20-25 | Total | PRE | 73 | 19 | 26.0 | 16.5 | 37.6 | 5.8 | 4.6 | 7.5 | <7.0 | 510.0 |
| | | | | M3 | 73 | 73 | 100 | 95.1 | 100 | 2743.4 | 2167.5 | 3472.3 | 122.0 | 19057.0 |
| | V40_06 | 9-14 | Total | PRE | 64 | 2 | 3.1 | 0.4 | 10.8 | 3.6 | 3.4 | 3.9 | <7.0 | 22.0 |
| | | | | M7 | 64 | 64 | 100 | 94.4 | 100 | 8085.8 | 6654.5 | 9825.0 | 1073.0 | 60059.0 |
| | | 15-19 | Total | PRE | 74 | 5 | 6.8 | 2.2 | 15.1 | 4.0 | 3.5 | 4.7 | <7.0 | 387.0 |
| | | | | M7 | 74 | 74 | 100 | 95.1 | 100 | 6170.1 | 5046.8 | 7543.5 | 412.0 | 91976.0 |
| | | 20-25 | Total | PRE | 68 | 15 | 22.1 | 12.9 | 33.8 | 5.4 | 4.3 | 6.9 | <7.0 | 329.0 |
| | | | | M7 | 68 | 68 | 100 | 94.7 | 100 | 4389.6 | 3525.6 | 5465.4 | 619.0 | 34350.0 |
| | V20_06 | 9-14 | Total | PRE | 69 | 5 | 7.2 | 2.4 | 16.1 | 3.8 | 3.5 | 4.1 | <7.0 | 44.0 |
| | | | | M7 | 69 | 69 | 100 | 94.8 | 100 | 5630.7 | 4772.1 | 6643.7 | 1094.0 | 36047.0 |
| | | 15-19 | Total | PRE | 69 | 6 | 8.7 | 3.3 | 18.0 | 4.2 | 3.6 | 4.8 | <7.0 | 76.0 |
| | | | | M7 | 69 | 69 | 100 | 94.8 | 100 | 5039.3 | 4283.4 | 5928.5 | 1406.0 | 34562.0 |
| | | 20-25 | Total | PRE | 66 | 17 | 25.8 | 15.8 | 38.0 | 5.5 | 4.4 | 7.0 | <7.0 | 141.0 |

| | | | | | | | | **95% Cl** | | | **95% Cl** | | | |
|---|---|---|---|---|---|---|---|---|---|---|---|---|---|---|
| **Antibody** | **Group** | **Sub-group** | **Pre-vacc status** | **Timing** | **N** | **n** | **%** | **LL** | **UL** | **value** | **LL** | **UL** | **Min** | **Max** |
| | | | | M7 | 66 | 66 | 100 | 94.6 | 100 | 3889.2 | 2980.9 | 5074.3 | 163.0 | 24791.0 |
| | HPV | 9-14 | Total | PRE | 75 | 7 | 9.3 | 3.8 | 18.3 | 4.0 | 3.6 | 4.5 | <7.0 | 43.0 |
| | | | | M7 | 75 | 75 | 100 | 95.2 | 100 | 7192.9 | 5952.6 | 8691.6 | 1313.0 | 37491.0 |
| | | 15-19 | Total | PRE | 66 | 5 | 7.6 | 2.5 | 16.8 | 3.8 | 3.5 | 4.2 | <7.0 | 17.0 |
| | | | | M7 | 66 | 66 | 100 | 94.6 | 100 | 4907.0 | 3780.8 | 6368.7 | 391.0 | 84753.0 |
| | | 20-25 | Total | PRE | 67 | 14 | 20.9 | 11.9 | 32.6 | 5.2 | 4.2 | 6.6 | <7.0 | 161.0 |
| | | | | M7 | 67 | 67 | 100 | 94.6 | 100 | 3576.8 | 2886.5 | 4432.2 | 709.0 | 48127.0 |

| | | | | | | | | | | | | | | |
|---|---|---|---|---|---|---|---|---|---|---|---|---|---|---|
| V40_02 = HPV-16/18(40,40) AS04 0,2 m V40_06 = HPV-16/18(40,40) AS04 0,6 m V20_06 = HPV-16/18(20,20) AS04 0,6 m HPV = HPV-16/18(20,20) AS04 0,1,6 m 9-14 = 9 to 14 years 15-19 = 15 to 19 years 20-25 = 20 to 25 years GMT = geometric mean antibody titer calculated on all subjects N = number of subjects with pre-vaccination results available n/% = number/percentage of subjects with titer within the specified range 95% CI = 95% confidence interval; LL = Lower Limit, UL = Upper Limit MIN/MAX = Minimum/Maximum PRE = Pre-vaccination | | | | | | | | | | | | | | |

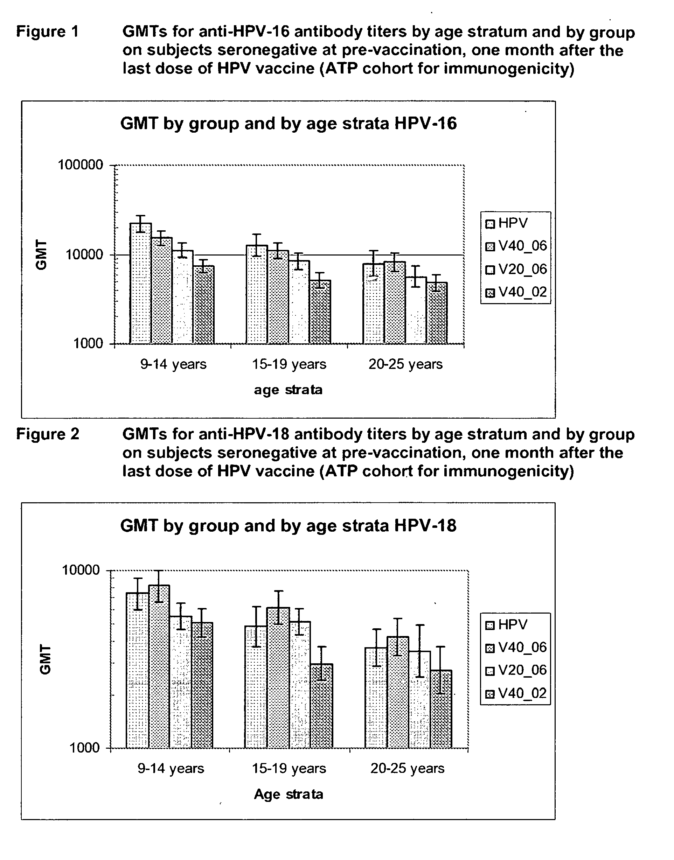

Figure 1 and Figure 2 illustrate the GMTs for anti-HPV-16 and anti-HPV 18 antibody titers one month after the last dose of HPV vaccine by age stratum and by group on subjects seronegative at pre-vaccination. For both antigens, there was a decrease in GMTs as a function of age, which was less pronounced for HPV-18 than for HPV-16.

### Inferential analyses

### 1. Primary immunogenicity objective

The primary objective of this study was to evaluate the immunogenicity of the HPV-16/18 L1 VLP AS04 vaccine one month after the last dose when administered at different dosages (20 or 40 µg of each HPV antigen) and on different schedules (0,2- or 0,6-months) compared with the standard HPV-16/18 L1 VLP AS04 vaccine administered on a 3-dose schedule (0,1,6-months).

### HPV-16

The two-way ANOVA model that was applied using titers (log₁₀) as response variable revealed that the group-by-age interaction was not statistically significant (p=0.195). The effect of group and age was significant (p<0.0001).

Pair wise comparisons were done between each 2-dose schedule group and the standard HPV-16/18 L1 VLP AS04 vaccine using Dunnett's tests. The standard HPV-16/18 L1 VLP AS04 vaccine was to be considered superior to a 2-dose formulation/schedule if the lower limit of the 95%CI was inferior to 0.5 (2-fold difference). The standard HPV-16/18 L1 VLP AS04 vaccine was found superior to the 40/40 M0,2 but not to 40/40 M0,6 and 20/20 M0,6 (Table 6).

Geometric mean ratios between each 2-dose schedule group and the standard HPV-16/18 L1 VLP AS04 vaccine group can be found in Table 7.

**Table 6 Pair wise comparisons between each 2-dose schedule group and the 3-dose standard schedule group for anti-HPV-16 antibody titers (ATP cohort for immunogenicity)**

| **GROUP** | **N** | **Adjusted GMT** | **LL** | **UL** | **GROUP** | **N** | **Adjusted GMT** | **LL** | **UL** | **GMR** | **LL** | **UL** |
|---|---|---|---|---|---|---|---|---|---|---|---|---|
| V40_02 | 201 | 5692.17 | 5148.24 | 6293.56 | HPV | 178 | 13164.78 | 11833.99 | 14645.23 | 0.43 | 0.36 | 0.52 |
| V40_06 | 173 | 11203.54 | 10049.40 | 12490.23 | HPV | 178 | 13164.78 | 11833.99 | 14645.23 | 0.85 | 0.70 | 1.03 |
| V20 06 | 178 | 8092.90 | 7275.41 | 9002.25 | HPV | 178 | 13164.78 | 11833.99 | 14645.23 | 0.61 | 0.51 | 0.74 |

| | | | | | | | | | | | | |
|---|---|---|---|---|---|---|---|---|---|---|---|---|
| V40_02 = HPV-16/18(40,40) AS04 0,2 m V40_06 = HPV-16/18(40,40) AS04 0,6 m V20_06 = HPV-16/18(20,20) AS04 0,6 m HPV = HPV-16/18(20,20) AS04 0,1,6 m GMR = Geometric Mean Ratio LL/UL = Lower and Upper Limits of the 95% confidence interval Adjusted GMT = GMT adjusted on age strata | | | | | | | | | | | | |

**Table 7 Geometric Mean Ratio between each 2-dose schedule group and the 3-dose standard schedule group for anti-HPV-16 antibody titers (ATP cohort for immunogenicity)**

| **GROUP** | **Age strata** | **N** | **GMT** | **LL** | **UL** | **GROUP** | **N** | **GMT** | **LL** | **UL** | **GMR** |
|---|---|---|---|---|---|---|---|---|---|---|---|
| V40_02 | 9-14 | 75 | 7441.87 | 6425.97 | 8618.37 | HPV | 67 | 22261.26 | 19059.36 | 26001.07 | 0.33 |
| V40_02 | 15-19 | 70 | 5153.28 | 4328.05 | 6135.85 | HPV | 60 | 12857.58 | 10648.67 | 15524.69 | 0.40 |
| V40_02 | 20-25 | 56 | 4809.14 | 3915.82 | 5906.24 | HPV | 51 | 7971.35 | 6427.10 | 9886.64 | 0.60 |
| V40_06 | 9-14 | 61 | 15304.16 | 13005.56 | 18009.02 | HPV | 67 | 22261.26 | 19059.36 | 26001.07 | 0.69 |
| V40_06 | 15-19 | 66 | 11060.88 | 9241.36 | 13238.65 | HPV | 60 | 12857.58 | 10648.67 | 15524.69 | 0.85 |
| V40_06 | 20-25 | 46 | 8307.43 | 6622.15 | 10421.61 | HPV | 51 | 7971.35 | 6427.10 | 9886.64 | 1.05 |
| V20_06 | 9-14 | 65 | 11066.95 | 9452.72 | 12956.84 | HPV | 67 | 22261.26 | 19059.36 | 26001.07 | 0.50 |
| V20_06 | 15-19 | 62 | 8442.27 | 7013.37 | 10162.29 | HPV | 60 | 12857.58 | 10648.67 | 15524.69 | 0.66 |
| V20_06 | 20-25 | 51 | 5673.17 | 4574.13 | 7036.27 | HPV | 51 | 7971.35 | 6427.10 | 9886.64 | 0.71 |

| | | | | | | | | | | | |
|---|---|---|---|---|---|---|---|---|---|---|---|
| V40_02 = HPV-16/18(40,40) AS04 0,2 m V40_06 = HPV-16/18(40,40) AS04 0,6 m V20_06 = HPV-16/18(20,20) AS04 0,6 m HPV = HPV-16/18(20,20) AS04 0,1,6 m GMR = Geometric Mean Ratio LL/UL = Lower and Upper Limits of the 95% confidence interval | | | | | | | | | | | |

### HPV-18

The two-way ANOVA model that was applied using titers (log₁₀) as response variable revealed that the group-by-age interaction was not statistically significant (p=0.435). The effect of group and age was significant (p<0.0001).

Pair wise comparisons were done between each 2-dose schedule group and the standard HPV-16/18 L1 VLP AS04 vaccine using Dunnett's tests. The standard HPV-16/18 L1 VLP AS04 vaccine was to be considered superior to a 2-dose formulation/schedule if the lower limit of the 95%CI was inferior to 0.5 (2-fold difference). The standard HPV-16/18 L1 VLP AS04 vaccine was not found superior to any of the three 2-dose groups (Table 8).

Geometric mean ratios between each 2-dose schedule group and the standard HPV-16/18 L1 VLP AS04 vaccine group can be found in Table 9.

**Table 8 Pair wise comparisons between each 2-dose schedule group and the 3-dose standard schedule group for anti-HPV-18 antibody titers (ATP cohort for immunogenicity)**

| **GROUP** | **N** | **Adjusted GMT** | **LL** | **UL** | **GROUP** | **N** | **Adjusted GMT** | **LL** | **UL** | **GMR** | **LL** | **UL** |
|---|---|---|---|---|---|---|---|---|---|---|---|---|
| V40_02 | 192 | 3468.22 | 3120.68 | 3854.46 | HPV | 182 | 5088.91 | 4566.64 | 5670.92 | 0.68 | 0.56 | 0.82 |
| V40_06 | 184 | 5968.26 | 5358.51 | 6647.39 | HPV | 182 | 5088.91 | 4566.64 | 5670.92 | 1.17 | 0.97 | 1.42 |
| V20_06 | 176 | 4638.79 | 4154.06 | 5180.09 | HPV | 182 | 5088.91 | 4566.64 | 5670.92 | 0.91 | 0.75 | 1.11 |

| | | | | | | | | | | | | |
|---|---|---|---|---|---|---|---|---|---|---|---|---|
| V40_02 = HPV-16/18(40,40) AS04 0,2 m V40_06 = HPV-16/18(40,40) AS04 0,6 m V20_06 = HPV-16/18(20,20) AS04 0,6 m HPV = HPV-16/18(20,20) AS04 0,1,6 m GMR = Geometric Mean Ratio LL/UL = Lower and Upper Limits of the 95% confidence interval Adjusted GMT = GMT adjusted on age strata | | | | | | | | | | | | |

**Table 9 Geometric Mean Ratio between each 2-dose schedule group and the 3-dose standard schedule group for anti-HPV-18 antibody titers (ATP cohort for immunogenicity)**

| **GROUP** | **Age strata** | **N** | **GMT** | **LL** | **UL** | **GROUP** | **N** | **GMT** | **LL** | **UL** | **GMR** |
|---|---|---|---|---|---|---|---|---|---|---|---|
| V40_02 | 9-14 | 70 | 5095.39 | 4370.25 | 5940.83 | HPV | 68 | 7398.84 | 6331.69 | 8645.84 | 0.69 |
| V40_02 | 15-19 | 68 | 2986.42 | 2505.14 | 3560.16 | HPV | 61 | 4845.03 | 4024.56 | 5832.77 | 0.62 |
| V40_02 | 20-25 | 54 | 2741.52 | 2187.37 | 3436.04 | HPV | 53 | 3676.34 | 2927.03 | 4617.47 | 0.75 |
| V40_06 | 9-14 | 62 | 8155.44 | 6927.98 | 9600.39 | HPV | 68 | 7398.84 | 6331.69 | 8645.84 | 1.10 |
| V40_06 | 15-19 | 69 | 6161.92 | 5175.50 | 7336.34 | HPV | 61 | 4845.03 | 4024.56 | 5832.77 | 1.27 |
| V40_06 | 20-25 | 53 | 4230.38 | 3368.14 | 5313.34 | HPV | 53 | 3676.34 | 2927.03 | 4617.47 | 1.15 |
| V20_06 | 9-14 | 64 | 5509.83 | 4692.59 | 6469.39 | HPV | 68 | 7398.84 | 6331.69 | 8645.84 | 0.74 |
| V20_06 | 15-19 | 63 | 5141.91 | 4283.86 | 6171.83 | HPV | 61 | 4845.03 | 4024.56 | 5832.77 | 1.06 |
| V20_06 | 20-25 | 49 | 3523.32 | 2779.73 | 4465.83 | HPV | 53 | 3676.34 | 2927.03 | 4617.47 | 0.96 |

| | | | | | | | | | | | |
|---|---|---|---|---|---|---|---|---|---|---|---|
| V40_02 = HPV-16/18(40,40) AS04 0,2 m V40_06 = HPV-16/18(40,40) AS04 0,6 m V20_06 = HPV-16/18(20,20) AS04 0,6 m HPV = HPV-16/18(20,20) AS04 0,1,6 m GMR = Geometric Mean Ratio LL/UL = Lower and Upper Limits of the 95% confidence interval | | | | | | | | | | | |

### 2. Secondary immunogenicity objectives

### HPV-16

For the 9-14 years stratum, 40/40 M0,2 group was non-inferior to the standard HPV-16/18 L1 VLP AS04 vaccine in subjects 15 - 25 years of age. There was no evidence of non-inferiority for the 15-19 years and 20-25 years strata in the 40/40 M0,2 group compared to the standard HPV-16/18 L1 VLP AS04 vaccine in subjects 15 - 25 years of age

For each age stratum, 40/40 M0,6 group was non-inferior to the standard HPV-16/18 L1 VLP AS04 vaccine in subjects 15 - 25 years of age.

For each age stratum, except for subjects aged 20 to 25 years, 20/20 M0,6 was non-inferior to the standard HPV-16/18 L1 VLP AS04 vaccine in subjects 15 - 25 years of age (Table 10 to Table 12).

**Table 10 Non-inferiority of the anti-HPV-16 titers response to the 2-dose schedule of the HPV-16/18 L1 VLP AS04 vaccine in the 9- 14 years of age stratum when administered at different dosages and on different schedules compared to the standard 3-dose schedule in subjects 15 - 25 years of age, one month after the last dose of active vaccine (ATP cohort for immunogenicity)**

| | | | | **GMT ratio (HPV / V40_02)** | | |
|---|---|---|---|---|---|---|
| **HPV** | | **V40_02** | | | **95% CI** | |
| **N** | **GMT** | **N** | **GMT** | **Value** | **LL** | **UL** |
| 111 | 10322.0 | 75 | 7441.9 | 1.39 | 1.03 | 1.87 |

| | | | | **GMT ratio (HPV / V40_06)** | | |
|---|---|---|---|---|---|---|
| **HPV** | | **V40_06** | | | **95% Cl** | |
| **N** | **GMT** | **N** | **GMT** | **Value** | **LL** | **UL** |
| 111 | 10322.0 | 61 | 15304.2 | 0.67 | 0.49 | 0.92 |

| | | | | **GMT ratio (HPV / V20_06)** | | |
|---|---|---|---|---|---|---|
| **HPV** | | **V20_06** | | | **95% Cl** | |
| **N** | **GMT** | **N** | **GMT** | **Value** | **LL** | **UL** |
| 111 | 10322.0 | 65 | 11066.9 | 0.93 | 0.68 | 1.28 |

| | | | | | | |
|---|---|---|---|---|---|---|
| V40_02 = HPV-16/18(40,40) AS04 0,2 m V40_06 = HPV-16/18(40,40) AS04 0,6 m V20_06 = HPV-16/18(20,20) AS04 0,6 m HPV = HPV-16/18(20,20) AS04 0,1,6 m GMT = geometric mean antibody titer N = Number of subjects with pre-vaccination results available 95% CI = 95% confidence interval for the GMT ratio (ANOVA model - pooled variance); LL = lower limit, UL = upper limit | | | | | | |

**Table 11 Non-inferiority of the anti-HPV-16 titers response to the 2-dose schedule of the HPV-16/18 L1 VLP AS04 vaccine in the 15- 19 years of age stratum when administered at different dosages and on different schedules as compared to the standard 3-dose schedule in subjects 15 - 25 years of age, one month after the last dose of active vaccine (ATP cohort for immunogenicity)**

| | | | | **GMT ratio (HPV / V40_02)** | | |
|---|---|---|---|---|---|---|
| **HPV** | | **V40_02** | | | **95% CI** | |
| **N** | **GMT** | **N** | **GMT** | **Value** | **LL** | **UL** |
| 111 | 10322.0 | 70 | 5153.3 | 2.00 | 1.47 | 2.73 |

| | | | | **GMT ratio (HPV** / **V40_06)** | | |
|---|---|---|---|---|---|---|
| **HPV** | | **V40_06** | | | **95% Cl** | |
| **N** | **GMT** | **N** | **GMT** | **Value** | **LL** | **UL** |
| 111 10322.0 | | 66 | 11060.9 | 0.93 | 0.68 | 1.28 |

| | | | | **GMT ratio (HPV / V20_06)** | | |
|---|---|---|---|---|---|---|
| **HPV** | | **V20_06** | | | **95% Cl** | |
| **N** | **GMT** | **N** | **GMT** | **Value** | **LL** | **UL** |
| 111 | 10322.0 | 62 | 8442.3 | 1.22 | 0.89 | 1.69 |

| | | | | | | |
|---|---|---|---|---|---|---|
| V40_02 = HPV-16/18(40,40) AS04 0,2 m V40_06 = HPV-16/18(40,40) AS04 0,6 m V20_06 = HPV-16/18(20,20) AS04 0,6 m HPV = HPV-16%18(20,20) AS04 0,1,6 m GMT = geometric mean antibody titer N = Number of subjects with pre-vaccination results available 95% CI = 95% confidence interval for the GMT ratio (ANOVA model - pooled variance) LL = lower limit, UL = upper limit | | | | | | |

**Table 12 Non-inferiority of the anti-HPV-16 titers response to the 2-dose schedule of the HPV-16/18 L1 VLP AS04 vaccine in the 20 - 25 years of age stratum when administered at different dosages and on different schedules as compared to the standard 3-dose schedule in subjects 15 - 25 years of age, one month after the last dose of active vaccine (ATP cohort for immunogenicity)**

| | | | | **GMT ratio (HPV/V40_02)** | | |
|---|---|---|---|---|---|---|
| **HPV** | | **V40_02** | | | **95% Cl** | |
| **N** | **GMT** | **N** | **GMT** | **Value** | **LL** | **UL** |
| 111 | 10322.0 | 56 | 4809.1 | 2.15 | 1.53 | 3.00 |

| | | | | **GMT ratio (HPV / V40_06)** | | |
|---|---|---|---|---|---|---|
| **HPV** | | **V40 06** | | | **95% Cl** | |
| **N** | **GMT** | **N** | **GMT** | **Value** | **LL** | **UL** |
| 111 | 10322.0 | 46 | 8307.4 | 1.24 | 0.86 | 1.79 |

| | | | | **GMT ratio (HPV / V20_06)** | | |
|---|---|---|---|---|---|---|
| **HPV** | | **V20_06** | | | **95% Cl** | |
| **N** | **GMT** | **N** | **GMT** | **Value** | **LL** | **UL** |
| 111 | 10322.0 | 51 | 5673.2 | 1.82 | 1.27 | 2.61 |

| | | | | | | |
|---|---|---|---|---|---|---|
| V40_02 = HPV-16/18(40,40) AS04 0,2 m V40_06 = HPV-16/18(40,40) AS04 0,6 m V20_06 = HPV-16/18(20,20) AS04 0,6 m HPV = HPV-16/18(20,20) AS04 0,1,6 m GMT = geometric mean antibody titer N = Number of subjects with pre-vaccination results available 95% CI = 95% confidence interval for the GMT ratio (ANOVA model - pooled variance) LL = lower limit, UL = upper limit | | | | | | |

### HPV-18

For each age stratum, each 2-dose formulation/schedule group was non-inferior to the standard HPV-16/18 L1 VLP AS04 vaccine in subjects 15 - 25 years of age, except for the 40/40 M0,2 group in subjects 20 - 25 years of age for which there was no evidence of non-inferiority (Table 13 to Table 15).

**Table 13 Non-inferiority of the anti-HPV-18 titers response to the 2-dose schedule of the HPV-16/18 L1 VLP AS04 vaccine in the 9- 14 years of age stratum when administered at different dosages and on different schedules as compared to the standard 3-dose schedule in subjects 15 - 25 years of age, one month after the last dose of active vaccine (ATP cohort for immunogenicity)**

| | | | | **GMT ratio (HPV / V40_02)** | | |
|---|---|---|---|---|---|---|
| **HPV** | | **V40_02** | | | **95% Cl** | |
| **N** | **GMT** | **N** | **GMT** | **Value** | **LL** | **UL** |
| 114 | 4261.5 | 70 | 5095.4 | 0.84 | 0.64 | 1.09 |

| | | | | **GMT ratio (HPV / V40_06)** | | |
|---|---|---|---|---|---|---|
| **HPV** | | **V40_06** | | | **95% CI** | |
| **N** | **GMT** | **N** | **GMT** | **Value** | **LL** | **UL** |
| 114 | 4261.5 | 62 | 8155.4 | 0.52 | 0.40 | 0.69 |

| | | | | **GMT ratio (HPV / V20_06)** | | |
|---|---|---|---|---|---|---|
| **HPV** | | **V20_06** | | | **95% CI** | |
| **N** | **GMT** | **N** | **GMT** | **Value** | **LL** | **UL** |
| 114 | 4261.5 | 64 | 5509.8 | 0.77 | 0.59 | 1.01 |

| | | | | | | |
|---|---|---|---|---|---|---|
| V40_02 = HPV-16/18(40,40) AS04 0,2 m V40_06 = HPV-16/18(40,40) AS04 0,6 m V20_06 = HPV-16/18(20,20) AS04 0,6 m HPV = HPV-16/18(20,20) AS04 0,1,6 m GMT = geometric mean antibody titer N = Number of subjects with pre-vaccination results available 95% CI = 95% confidence interval for the GMT ratio (ANOVA model - pooled variance) LL = lower limit, UL = upper limit | | | | | | |

**Table 14 Non-inferiority of the anti-HPV-18 titers response to the 2-dose schedule of the HPV-16/18 L1 VLP AS04 vaccine in the 15 - 19 years of age stratum when administered at different dosages and on different schedules as compared to the standard 3-dose schedule in subjects 15 - 25 years of age, one month after the last dose of active vaccine (ATP cohort for immunogenicity)**

| | | | | **GMT ratio (HPV / V40_02)** | | |
|---|---|---|---|---|---|---|
| **HPV** | | **V40_02** | | | **95% CI** | |
| **N** | **GMT** | **N** | **GMT** | **Value** | **LL** | **UL** |
| 114 | 4261.5 | 68 | 2986.4 | 1.43 | 1.07 | 1.90 |

| | | | | **GMT ratio (HPV / V40_06)** | | |
|---|---|---|---|---|---|---|
| **HPV** | | **V40_06** | | | **95% CI** | |
| **N** | **GMT** | **N** | **GMT** | **Value** | **LL** | **UL** |
| 114 | 4261.5 | 69 | 6161.9 | 0.69 | 0.52 | 0.91 |

| | | | | **GMT ratio (HPV/V20_06)** | | |
|---|---|---|---|---|---|---|
| **HPV** | | **V20_06** | | | **95% CI** | |
| **N** | **GMT** | **N** | **GMT** | **Value** | **LL** | **UL** |
| 114 | 4261.5 | 63 | 5141.9 | 0.83 | 0.64 | 1.08 |

| | | | | | | |
|---|---|---|---|---|---|---|
| V40_02 = HPV-16/18(40,40) AS04 0,2 m V40_06 = HPV-16/18(40,40) AS04 0,6 m V20_06 = HPV-16/18(20,20) AS04 0,6 m HPV = HPV-16/18(20,20) AS04 0,1,6 m GMT = geometric mean antibody titer N = Number of subjects with pre-vaccination results available 95% CI = 95% confidence interval for the GMT ratio (ANOVA model - pooled variance) LL = lower limit, UL = upper limit | | | | | | |

**Table 15 Non-inferiority of the anti-HPV-18 titers response to the 2-dose schedule of the HPV-16/18 L1 VLP AS04 vaccine in the 20 - 25 years of age stratum when administered at different dosages and on different schedules as compared to the standard 3-dose schedule in subjects 15 - 25 years of age, one month after the last dose of active vaccine (ATP cohort for immunogenicity)**

| | | | | **GMT ratio (HPV / V40_02)** | | |
|---|---|---|---|---|---|---|
| **HPV** | | **V40_02** | | | **95% CI** | |
| **N** | **GMT** | **N** | **GMT** | **Value** | **LL** | **UL** |
| 114 | 4261.5 | 54 | 2741.5 | 1.55 | 1.12 | 2.15 |

| | | | | **GMT ratio (HPV / V40_06)** | | |
|---|---|---|---|---|---|---|
| **HPV** | | **V40_06** | | | **95% CI** | |
| **N** | **GMT** | **N** | **GMT** | **Value** | **LL** | **UL** |
| 114 | 4261.5 | 53 | 4230.4 | 1.01 | 0.74 | 1.36 |

| | | | | **GMT ratio (HPV / V20_06)** | | |
|---|---|---|---|---|---|---|
| **HPV** | | **V20_06** | | | **95% CI** | |
| **N** | **GMT** | **N** | **GMT** | **Value** | **LL** | **UL** |
| 114 | 4261.5 | 49 | 3523.3 | 1.21 | 0.86 | 1.71 |

| | | | | | | |
|---|---|---|---|---|---|---|
| V40_0L = HPV-16/18(40,40) AS04 0,2 m V40_06 = HPV-16/18(40,40) AS04 0,6 m V20_06 = HPV-16/18(20,20) AS04 0,6 m HPV = HPV-16/18(20,20) AS04 0,1,6 m GMT = geometric mean antibody titer N = Number of subjects with pre-vaccination results available 95% CI = 95% confidence interval for the GMT ratio (ANOVA model - pooled variance) LL = lower limit, UL = upper limit | | | | | | |

### Overall Conclusions:

A total of 960 subjects were vaccinated in this study (240 subjects in the 40/40 M0,2 group, 241 subjects in the 40/40 M0,6 group, 240 subjects in the 20/20 M0,6 group and 239 subjects in the standard

HPV-16/18 L1 VLP AS04 group). They were 17.2 ± 4.3 years-old (mean ± SD) on average. The majority of them were White Caucasian/European Heritage (96.7%).

All subjects in all groups were seropositive one month after the full vaccination course (at Month 3 [one month post-dose II in the 40/40 M0,2 group] and Month 7 [one month post-dose II in the 40/40 M0,6 and 20/20 M0,6 groups and one month post-dose III in the HPV group]). All subjects were also seropositive at Month 3, one month post-dose I in the 40/40 M0,6 and 20/20 M0,6 groups.

For both antigens, the age-group interaction was not statistically significant in the ATP cohort. For HPV-16, the standard HPV-16/18 L1 VLP AS04 vaccine was superior to the 40/40 M0,2 but not to the 40/40 M0,6 and 20/20 M0,6. For HPV-18, the standard HPV-16/18 L1 VLP AS04 vaccine was not superior to any of the three 2-dose groups.

With respect to the HPV-16 response:
- For each age stratum, the 40/40 M0,6 group was non-inferior to the standard HPV-16/18 L1 VLP AS04 vaccine in subjects 15 - 25 years of age.
- For each age stratum, the 20/20 M0,6 group was non-inferior to the standard HPV-16/18 L1 VLP AS04 vaccine in subjects 15 - 25 years of age, except for subjects aged 20 to 25 years.
- The 40/40 M0,2 group was non-inferior to the standard HPV-16/18 L1 VLP AS04 vaccine in subjects 15 - 25 years of age for the 9-14 years stratum only; there was no evidence of non-inferiority for the 15-19 years and 20-25 years strata in the 40/40 M0,2 group compared to the standard HPV-16/18 L1 VLP AS04 vaccine in subjects 15 - 25 years of age.

With respect to the HPV-18 response: for each age stratum, each 2-dose schedule group was non-inferior to the standard HPV-16/18 L1 VLP AS04 vaccine in subjects 15 - 25 years of age, except for the 40/40 M0,2 group in subjects 20 - 25 years of age for which there was no evidence of non-inferiority.

### Example 3 - Comparison of the immunogenicity of CervarixTM (trademark of the GlaxoSmithKline group of companies) and Gardasil (registered trademark of Merck & Co Inc)

Two prophylactic HPV vaccines have recently been licensed in many countries. Both use virus-like particles (VLPs) of recombinant L1 capsid proteins of individual HPV types to prevent HPV-16 and -18 cervical precancerous lesions and cancers. *Cervarix™* contains HPV-16 and -18 VLPs produced in *Trichoplusia* ni Rix4446 cell substrate using a baculovirus expression vector system and formulated with the proprietary immunostimulatory Adjuvant System 04 (AS04; comprised of 3-O-desacyl-4'-monophosphoryl lipid A [MPL] and aluminum hydroxide salt). *Gardasil®* contains HPV-16 and -18 VLPs produced in the yeast *Saccharomyces cerevisiae* and formulated with amorphous aluminum hydroxyphosphate sulfate salt. In addition, *Gardasil^{®}* contains VLPs from non-oncogenic types HPV-6 and -11, which are implicated in 75-90% of genital warts. For both vaccines, protection against infection with oncogenic types HPV-16 and HPV-18 and associated precancerous lesions has been demonstrated in randomized clinical trials. Protection has been demonstrated for at least 6.4 years post-vaccination for *Cervarix™* and at least 5 years for *Gardasil^{®}.*

This randomized, observer-blind study compared the two vaccines in a single, well-defined population of healthy women aged 18-45 years, using identical methodology for assessment of immunogenicity and safety. *Cervarix™* and *Gardasil^{®}* were administered according to their recommended three-dose vaccination schedules (Months 0,1, 6 and Months 0, 2, 6, respectively). The age range of 18-45 years was chosen to enable full characterization of the immune response to vaccination. This age range also provides stringent conditions for comparison of the two vaccines, as immune response to vaccination decreases with increasing age. Neutralizing antibody levels induced by the two vaccines were evaluated using a psuedovirus-based neutralization assay (PBNA) assay (see Harper et al. Lancet 2004; 364(9447):1757-65 and Harper et al. Lancet 2006; 367(9518):1247-55) in order to objectively compare functional immune responses using an unbiased assay.

A total of 1106 women were enrolled and vaccinated; 553 in each group. The vaccines and administration schedules are shown in Table 17.

Seropositivity rates and geometric mean titers (GMTs) for HPV-16 and HPV-18 antibodies, measured by PBNA for immunogenicity on women who were seronegative and deoxyribonucleic acid (DNA) negative prior to vaccination for the HPV antigen under analysis, are shown by age stratification in Table 16. One month after completion of the three-dose vaccination course (Month 7), all women in both vaccine groups had seroconverted for HPV-16 and HPV-18, except for two women aged 27-35 years in the *Gardasil^{®}* group who did not seroconvert for HPV-18. Table 16 also shows results at Month 6 i.e. after the second dose of vaccine.

For all age groups combined, neutralizing antibody GMTs measured by PBNA in women in the total vaccinated cohort who had cleared natural infection (i.e., seropositive and DNA negative at Month 0 for the HPV antigen under analysis) were 180.1 ED₅₀ (effective dose producing 50% response) [95% confidence interval (CI): 153.3, 211.4] for HPV-16 and 137.3 ED₅₀ [95% CI: 112.2, 168.0] for HPV-18. For both vaccines, neutralizing antibody GMTs at Month 7 in women in the ATP cohort for immunogenicity who were seronegative and DNA negative prior to vaccination for the HPV antigen under analysis (Table 16) were well above those associated with natural infection. Non-inferiority of HPV-16 and -18 immune responses of *Cervarix*™ versus *Gardasil^{®}* was shown in all three age groups for both HPV-16 and HPV-18 (Table 16). Anti-HPV-16 and -18 neutralizing antibody GMTs at Month 7 were 3.7- and 7.3-fold higher, respectively, in the *Cervarix*™ group than in the *Gardasil^{®}* group in women aged 18-26 years (Table 16). Compared with *Gardasil^{®},* anti-HPV-16 and -18 GMTs with *Cervarix*™ were 4.8- and 9.1-fold higher in women aged 27-35 years and 2.3- and 6.8-fold higher in women aged 36-45 years, respectively (Table 16). Analysis of antibody kinetics before dose three (Month 6) showed that anti-HPV-18 antibody levels were already higher in the *Cervarix*™ group than in the *Gardasil^{®}* group after two vaccine doses; the lower limit of the two-sided 97.6% CI for the GMT ratio was >1 in all age groups (Table 16). No differences in anti-HPV-16 GMTs were seen between the two vaccine groups prior to dose three (Table 16).

Superiority testing performed on the total vaccinated cohort (irrespective of HPV serostatus and HPV DNA status prior to vaccination) confirmed the neutralizing antibody levels induced by *Cervarix™* to be significantly higher than that induced by *Gardasil^{®}* for each antigen in all age groups (p<0.0001).

**Table 16 Seropositivity rates, GMTs and GMT ratios for HPV-16 and HPV-18 serum neutralizing antibodies measured by pseudovirion-based neutralization assay at Months 6 and 7 (seronegative and DNA negative prior to vaccination)**

| ***(a)* 18-26 *years*** | | | | | | | | | |
|---|---|---|---|---|---|---|---|---|---|
| | | ***Cervarix*™** | | | ***Gardasil^{®}*** | | | | |
| **Antigen** | **Month** | **N** | **% SP [95% CI]** | **GMT [95% CI]** | **N** | **% SP [95% CI]** | **GMT [95% CI]** | **GMT ratio** | **97.6% CI** |
| **HPV-16** | 6 | 104 | 100 [96.5, 100] | 1628 [1304,2032] | 102 | 99.0 [94.7, 100] | 1592 [1204, 2106] | 1.0 | 0.7, 1.5 |
| | 7 | 104 | 100 [96.5, 100] | 36792 [29266, 46254] | 103 | 100 [96.5, 100] | 10053 [8136, 12422] | 3.7 | 2.6, 5.2 |
| **HPV-18** | 6 | 118 | 99.2 [95.4, 100] | 686 [549, 858] | 130 | 93.1 [87.3, | 234 [187, 294] | 2.9 | 2.0, 4.2 |
| | 7 | 118 | 100 [96.9, 100] | 16487 [13384, 20310] | 131 | 96.8] 100 [97.2, 100] | 2258 [1809, 2818] | 7.3 | 5.1, 10.4 |

| ***(b)* 27-35 *years*** | | | | | | | | | |
|---|---|---|---|---|---|---|---|---|---|
| | | ***Cervarix*™** | | | ***Gardasil^{®}*** | | | | |
| **Antigen** | **Month** | **N** | **% SP [95% CI]** | **GMT [95% CI]** | **N** | **% SP [95% CI]** | **GMT [95% CI]** | **GMT ratio** | **97.6% CI** |
| **HPV-16** | 6 | 90 | 100 [96.0,100] | 1263 [893, 1787] | 84 | 98.8 [93.5, 100] | 1014 [738, 1394] | 1.2 | 0.7, 2.1 |
| | 7 | 90 | 100 [96.0,100] | 23908 [18913, 30222] | 85 | 100 [95.8, 100] | 4958 [3896, 6311] | 4.8 | 3.3, 7.1 |
| **HPV-18** | 6 | 102 | 97.1 [91.6,99.4] | 429 [326, 564] | 100 | 84.0 [75.3,90.6] | 176 [133, 233] | 2.4 | 1.6, 3.8 |
| | 7 | 102 | 100 [96.4, 100] | 9502 [7519, 12008] | 101 | 98.0 [93.0, 99.8] | 1043 [790, 1378] | 9.1 | 6.0, 13.8 |

| ***(c) 36-45 years*** | | | | | | | | | |
|---|---|---|---|---|---|---|---|---|---|
| | | ***Cervarix*™** | | | ***Gardasil*^{®}** | | | | |
| **Antigen** | **Month** | **N** | **% SP [95% CI]** | **GMT [95% CI]** | **N** | **% SP [95% CI]** | **GMT [95% CI]** | **GMT ratio** | **97.6% CI** |
| **HPV-16** | 6 | 96 | 99.9 [94.3, 100] | 1730 [1215, 2463] | 81 | 100 [95.5, 100] | 1917 [1361, 2698] | 0.9 | 0.5, 1.6 |
| | 7 | 96 | 100 [96.2, 100] | 17302 [13605, 22002] | 83 | 100 [95.7, 100] | 7634 [5916, 9853] | 2.3 | 1.5, 3.4 |
| **HPV-18** | 6 | 110 | 97.3 [92.2,99.4] | 619 [447, 857] | 89 | 87.6 [79.0,93.7] | 169 [127, 224] | 3.7 | 2.2, 6.1 |
| | 7 | 110 | 100 [96.7, 100] | 9846 [7835, 12372] | 91 | 100 [96.0, 100] | 1439 [1105, 1873] | 6.8 | 4.6, 10.2 |

| | | | | | | | | | |
|---|---|---|---|---|---|---|---|---|---|
| GMT, geometric mean antibody titer; SP, seropositivity (defined as neutralizing antibody titer ≥40 ED₅₀) GMT ratio = *Cervarix*™ GMT divided by *Gardasil*^{®} GMT at Month 6 and Month 7 computed using an ANOVA model on the log₁₀ transformation of the titers in each age cohort | | | | | | | | | |

**Table 17 Composition of the study vaccines and administration schedules**

| | ***Cervarix™*** | ***Gardasil^{®}*** |
|---|---|---|
| **Antigens** | 20 µg HPV-16 VLP | 40 µg HPV-16 VLP |
| | 20 µg HPV-18 VLP | 20 µg HPV-18 VLP |
| | | 20 µg HPV-6 VLP |
| | | 40 µg HPV-11 VLP |
| **Expression system** | Baculovirus expression vector system in *Trichoplusia ni* Rix4446 cell substrate | *Saccharomyces cerevisiae* yeast |
| **Adjuvant** | AS04 [50 µg MPL and 500 µg Al(OH)₃] | 225 µg amorphous aluminum hydroxyphosphate sulfate |
| **Administration schedule** | | |
| **Month 0** | *Cervarix*™ | *Gardasil^{®}* |
| **Month 1** | *Cervarix*™ | Placebo [500 µg Al(OH)₃] |
| **Month 2** | Placebo [500 µg Al(OH)₃] | *Gardasil^{®}* |
| **Month 6** | *Cervarix*™ | *Gardasil^{®}* |

| | | |
|---|---|---|
| MPL, 3-O-desacyl-4'-monophosphoryl lipid A | | |

## Claims

1. A composition comprising HPV 16 and HPV 18 virus like particles (VLPs) together with a pharmaceutically acceptable excipient, for use as a vaccine in the prevention of human papillomavirus related disease or infection in a subject wherein the vaccine is formulated for administration to a subject 14 years of age or below according to a two dose regimen consisting of a first dose and a second dose.

2. A composition for use according to claim 1 wherein the second dose is to be administered 2 or 3 months after the first dose.

3. A composition for use according to claim 1 wherein the second dose is to be administered more than two months after the first dose.

4. A composition for use according to claim 3 wherein the second dose is to be administered 6 months after the first dose.

5. A composition for use according to any of claims 1 to 4 wherein the vaccine further comprises an adjuvant.

6. A composition for use according to claim 5 wherein the adjuvant comprises an aluminium salt.

7. A composition for use according to claim 6 wherein the aluminium salt is aluminium hydroxide.

8. A composition for use according to any of claims 5 to 7 wherein the adjuvant comprises a lipid A derivative.

9. A composition for use according to claim 8 wherein the adjuvant comprises 3D-MPL and aluminium hydroxide.

10. A composition for use according to any preceding claim wherein the 2 doses each comprise more than 20 µg of each of HPV 16 VLPs and HPV 18 VLPs, or 20 µg or 40 µg of each of HPV 16 VLPs and HPV 18 VLPs.

11. A composition for use according to any preceding claim wherein the VLPs comprise L1.

12. A composition for use according to any preceding claim wherein only VLPs of HPV types 16 and 18 are present in the vaccine.

13. A composition for use according to any preceding claim wherein the vaccine is for the prevention of human papillomavirus related disease or infection in females.

## Patentansprüche

1. Zusammensetzung, die HPV 16- und HPV 18-virusähnliche Partikel (VLPs) zusammen mit einem pharmazeutisch annehmbaren Träger umfasst, zur Verwendung als Vakzin zur Prävention einer Krankheit oder Infektion, die mit humanem Papillomavirus verbunden ist, bei einem Individuum, wobei das Vakzin zur Verabreichung an ein 14-jähriges oder jüngeres Individuum, gemäß eines 2-Gaben-Regimes, das aus einer ersten Gabe und einer zweiten Gabe besteht, formuliert ist.

2. Zusammensetzung zur Verwendung gemäß Anspruch 1, wobei die zweite Gabe 2 oder 3 Monate nach der ersten Gabe verabreicht werden soll.

3. Zusammensetzung zur Verwendung gemäß Anspruch 1, wobei die zweite Gabe mehr als 2 Monate nach der ersten Gabe verabreicht werden soll.

4. Zusammensetzung zur Verwendung gemäß Anspruch 3, wobei die zweite Gabe 6 Monate nach der ersten Gabe verabreicht werden soll.

5. Zusammensetzung zur Verwendung gemäß einem der Ansprüche 1 bis 4, wobei das Vakzin weiterhin ein Adjuvans umfasst.

6. Zusammensetzung zur Verwendung gemäß Anspruch 5, wobei das Adjuvans ein Aluminiumsalz umfasst.

7. Zusammensetzung zur Verwendung gemäß Anspruch 6, wobei das Aluminiumsalz Aluminiumhydroxid ist.

8. Zusammensetzung zur Verwendung gemäß einem der Ansprüche 5 bis 7, wobei das Adjuvans ein Lipid A-Derivat umfasst.

9. Zusammensetzung zur Verwendung gemäß Anspruch 8, wobei das Adjuvans 3D-MPL und Aluminiumhydroxid umfasst.

10. Zusammensetzung zur Verwendung gemäß einem der vorherigen Ansprüche, wobei die 2 Gaben jeweils mehr als 20 µg von jedem von HPV 16-VLPs und HPV 18-VLPs, oder 20 µg oder 40 µg von jedem von HPV 16-VLPs und HPV 18-VLPs umfassen.

11. Zusammensetzung zur Verwendung gemäß einem der vorherigen Ansprüche, wobei die VLPs L1 umfassen.

12. Zusammensetzung zur Verwendung gemäß einem der vorherigen Ansprüche, wobei nur VLPs von den HPV-Typen 16 und 18 in dem Vakzin vorhanden sind.

13. Zusammensetzung zur Verwendung gemäß einem der vorherigen Ansprüche, wobei das Vakzin zur Prävention einer Krankheit oder Infektion, die mit humanem Papillomavirus verbunden ist, bei weiblichen Individuen ist.

## Revendications

1. Composition comprenant des particules pseudovirales (VLP) du HPV 16 et du HPV 18 conjointement avec un excipient pharmaceutiquement acceptable pour une utilisation en tant que vaccin dans la prévention d'une maladie ou d'une infection associée aux papillomavirus humains chez un sujet, où le vaccin est formulé pour une administration à un sujet âgé de 14 ans ou moins selon un régime de deux doses consistant en une première dose et une seconde dose.

2. Composition pour une utilisation selon la revendication 1, où la seconde dose doit être administrée 2 ou 3 mois après la première dose.

3. Composition pour une utilisation selon la revendication 1, où la seconde dose doit être administrée plus de deux mois après la première dose.

4. Composition pour une utilisation selon la revendication 3, où la seconde dose doit être administrée 6 mois après la première dose.

5. Composition pour une utilisation selon l'une quelconque des revendications 1 à 4, où le vaccin comprend en outre un adjuvant.

6. Composition pour une utilisation selon la revendication 5, dans laquelle l'adjuvant comprend un sel d'aluminium.

7. Composition pour une utilisation selon la revendication 6, dans laquelle le sel d'aluminium est l'hydroxyde d'aluminium.

8. Composition pour une utilisation selon l'une quelconque des revendications 5 à 7, dans laquelle l'adjuvant comprend un dérivé du lipide A.

9. Composition pour une utilisation selon la revendication 8, dans laquelle l'adjuvant comprend du 3D-MPL et de l'hydroxyde d'aluminium.

10. Composition pour une utilisation selon l'une quelconque des revendications précédentes, où les 2 doses comprennent chacune plus de 20 µg de chacune des VLP du HPV 16 et des VLP du HPV 18, ou 20 µg ou 40 µg de chacune des VLP du HPV 16 et des VLP du HPV 18.

11. Composition pour une utilisation selon l'une quelconque des revendications précédentes, dans laquelle les VLP comprennent de la L1.

12. Composition pour une utilisation selon l'une quelconque des revendications précédentes, dans laquelle seules des VLP des types 16 et 18 de HPV sont présentes dans le vaccin.

13. Composition pour une utilisation selon l'une quelconque des revendications précédentes, où le vaccin est destiné à la prévention d'une maladie ou d'une infection associée aux papillomavirus humains chez des femmes.
